# EUROPEAN PATENT SPECIFICATION

(11) **EP 4 620 378 B1**
(45) Date of publication and mention of the grant of the patent: **02.09.2026**
(21) Application number: 24164461.6
(22) Date of filing: 19.03.2024
(51) Int. Cl.: A61B 3/00, A61B 3/10, A61B 3/13

(54) **CONTROL OF A HEIGHT-ADJUSTABLE PATIENT INTERFACE FOR AN OPHTHALMIC IMAGING DEVICE**
STEUERUNG EINER HÖHENVERSTELLBAREN PATIENTENSCHNITTSTELLE FÜR EINE OPHTHALMISCHE BILDGEBUNGSVORRICHTUNG
COMMANDE D'UNE INTERFACE PATIENT RÉGLABLE EN HAUTEUR POUR UN DISPOSITIF D'IMAGERIE OPHTALMIQUE

(43) Date of publication of application: 24.09.2025
(60) Divisional application: 26193011.9 / 4 799 553
(73) Proprietor: Optos plc, Dunfermline, Scotland KY11 8GR (GB)
(72) Inventor: BOLAND, Estelle, Dunfermline, Scotland KY11 8GR (GB); GOURLAY, Iain, Dunfermline, Scotland KY11 8GR (GB); ANDERSON, Alan, Dunfermline, Scotland KY11 8GR (GB); MUYO, Gonzalo, Dunfermline, Scotland KY11 8GR (GB); SUTTIE, Alan, Glasgow, G4 9SS (GB); SHEARLAW, Morven, Glasgow, G4 9SS (GB); SLORACH, Louis, Glasgow, G4 9SS (GB); LOUDON, Brian, Glasgow, G4 9SS (GB)
(74) Representative: Hoffmann Eitle

(56) References cited:
- EP-A1- 3 050 495
- EP-A1- 3 912 543
- JP-A- 2019 533 483
- US-A1- 2009 168 021
- US-A1- 2021 186 319
- US-B2- 7 792 249

## Description

### [Field]

Example aspects herein generally relate to the field of ophthalmic imaging devices and, in particular, to mechanisms for adjusting the height of a patient interface of an ophthalmic imaging device, such as a chinrest, a head rest, eyecup(s) or the like, which the patient's head contacts to steady the head during imaging.

### [Background]

Ophthalmic imaging devices employ various imaging techniques to image different parts of the eye, and are used by clinicians to diagnose and manage a variety of eye conditions. Ophthalmic imaging devices include (but are not limited to) scanning laser ophthalmoscopes (SLOs), optical coherence tomography (OCT) imaging devices, fundus cameras, microperimetry devices, and corneal topography devices (among others), or a combination of two or more such devices. Ophthalmic imaging devices typically include a height-adjustable patient interface (also referred to as a facial interface), such as a chinrest, a headrest, or an eyecup, for example, which has a contact surface that the patient's head contacts during imaging of the patient's eye. Such contact surfaces allow the location of the eye to be set to approximately the correct position with respect to the ophthalmic imaging device, and help keep the subject's head steady during the imaging, thereby reducing artefacts in the acquired images caused by movements of the patient's head relative to the device.

As subjects to be imaged by an ophthalmic imaging device will generally have a range of differing heights, the height of the patient interface is usually adjusted to suit each subject to be imaged. Adjusting the patient interface to an appropriate height for the subject is important, particularly for ophthalmic imaging devices such as microperimeters and optical coherence tomography (OC) imaging devices, for example, that require relatively long imaging sessions which can last from tens of seconds to several minutes (depending on the device), where movements of the eye during imaging may result in motion artifacts in the acquired ocular image. If the patient interface has not been set to a height which is comfortable for the subject, the subject will have a greater tendency to move during the imaging and thereby exacerbate the problem of imaging artefacts in the acquired image. This tends to be particularly problematic for older patients, who typically have a relatively limited range of positions that they can adopt comfortably enough to hold for the duration of the imaging session. If an uncomfortable position is set for an older patient, they may even fail to engage with the patient interface properly and have their eyes imaged at all.

Adjustment of the height of an ophthalmic imaging device's patient interface (so-called "macro alignment") is usually performed manually, by the operator monitoring the subject's eyeline whilst adjusting the height of the patient interface using a mechanical or electromechanical mechanism to approach the height of the eyeline. This is a time-consuming process, and one which usually needs to be repeated for every subject the operator is required to process. This adjustment may be particularly time-consuming and challenging to perform correctly for operators having limited skill and/or experience. Once the macro-alignment has been completed and the subject has engaged with the patient interface, the ophthalmic imaging device may perform an automatic micro-alignment process to align its scan head to the subject's eye.

Further background is provided in the following documents.

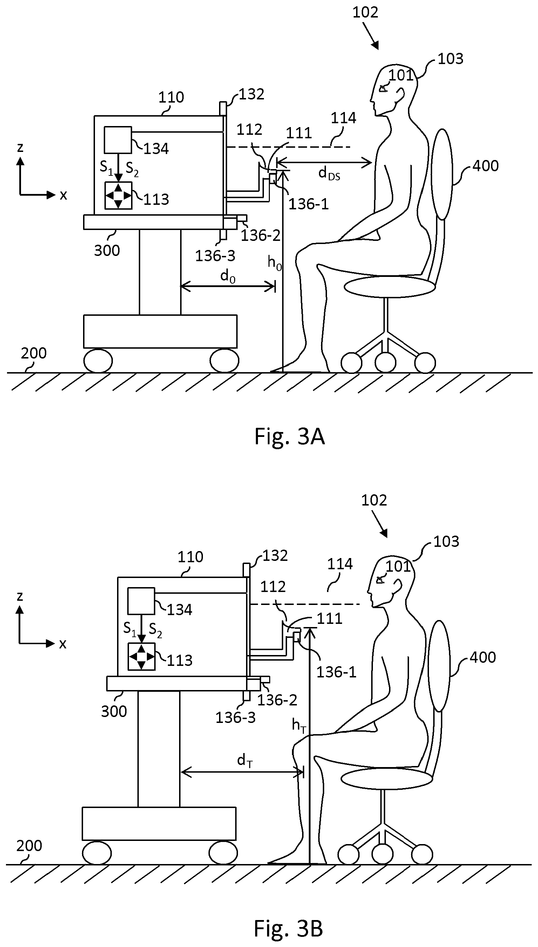

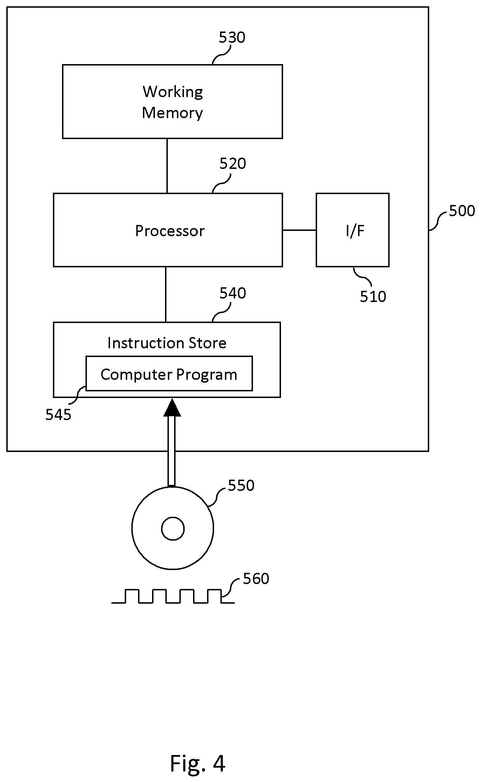

EP 3 050 495 A1 discloses a system having patient interface and a measurement tool for measuring an eye, such as an Optical Coherence Tomographer, that are supported on a table. The patient interface includes a height adjuster, a chin rest, and forehead rest. The adjuster mechanism may include adjusters operable to move the measurement tool and/or the optics in the X, Y, and Z directions relative to the patient interface. Scan positions 300a and 300b shown in Fig. 3A of EP 3 050 495 A1 may define the lower and upper limits of the scan range in a Y direction. The chin rest may be arranged for defining and maintaining a lateral facial measurement position with respect to measurement tool. A pre-scan position of the optics may capture an image that covers a facial area having a lateral position and lateral width appropriate to accommodate statistical variations of a pupillary distance from a vertical center axis passing through the centering rest portion. Thus, the patient and the optics are arranged such that the field-of-view (FOV) is as wide as or wider than pupillary bands 310a and 310b in Fig. 3A during pupil detection. Taking these parameters into account, the optics may operate from a predefined pre-scan position, as shown in Figs. 3A and 3B, corresponding to scan position 300b and scan towards scan position 300a until an eye is detected. The objective is adjusted for the detected eye via the adjuster mechanism. The first scan position is then recorded, which may be expressed as XY coordinates within an XY space. Thus, the recorded scan position may be the scan position of an objective of the optics upon pupil detection or the scan position after focusing. One or more measurements of the first eye via the optical tool in the first scan position are then triggered, and a rest position is recorded as a scalar value or a value within an XY or XYZ coordinate system. Some aspects of the control of the system are further described in paragraphs [0044] to [0054] with reference to Figs. 4A to 4C of EP 3 050 495 A1.

EP 3 912 543 A1 discloses an ophthalmic unit comprising a chair for a patient and an instrument-carrying table carrying an ophthalmic instrument and a support device for supporting the head of the patient. The chair and the table are each supported so as to be vertically adjustable between a respective position of minimum height from the ground and a respective position of maximum height from the ground. The ophthalmic unit is provided with an automatic positioning system for automatically adjusting the vertical position of the chair relative to the table. The automatic positioning system comprises a first linear actuation device arranged to vertically move the chair, a second linear actuation device arranged to vertically move the table, an electronic control unit arranged to automatically control, according to predetermined operating logics, the first linear actuation device and/or the second linear actuation device to adjust the vertical position of the chair relative to the table, and a first presence sensor arranged to provide the electronic control unit with information relating to the presence of the patient sitting on the chair at a given first height from the ground.

US 2009/168021 A discloses an opthalmologic apparatus having an examination unit including a plurality of examining parts for executing different examinations on an eye to and a driver moving the examination unit in the horizontal direction and in the anteroposterior direction. The opthalmologic apparatus controls the driver when switching a examination subject by the examination unit from one eye to the other eye to cause the examination unit to retreat by only a retreating distance according to the working distance of an examination part positioned at the examination position for the one eye. The examination unit retreats by only a distance according to the working distance. Therefore, it is possible to achieve both reductions in examination time and improvements for safety by preventing an examining part comes into contact with the patient.

### [Summary]

There is provided, in accordance with a first example aspect herein, a control system arranged to generate a control signal for adjusting a height at which a height-adjustable contact surface of an ophthalmic imaging device is disposed to a target height at which the contact surface is to contact a head of a subject during imaging of an eye of the subject by the ophthalmic imaging device. The control system comprises at least one camera arranged to acquire one or more images of at least a portion of the head of the subject where the subject is in a position beside the ophthalmic imaging device for the head to be brought into contact with the contact surface when the contact surface is at the target height and a processor. The control system further comprises a processor, which is arranged to: process the one or more images to generate a value of a first indicator which is indicative of a height at which the at least a portion of the head was disposed when the one or more images were acquired; use at least one mapping to map the value of the first indicator to a value of a second indicator which is indicative of the target height at which the contact surface is to contact the head during the imaging; and generate a control signal for adjusting the height at which the contact surface is disposed to the target height indicated by the value of the second indicator.

In an example embodiment, the height of the contact surface is adjustable by a user of the control system, and the control system further comprises a user interface for providing the user with instructions for adjusting the height of the contact surface. The user interface may comprise at least one of a display for providing the user with visual instructions for adjusting the height of the contact surface, and a speaker for providing the user with audio instructions for adjusting the height of the contact surface, for example. In the example embodiment, the processor is arranged to control the user interface, using the generated control signal, to provide the user with instructions for adjusting the height of the contact surface to the target height.

In another example embodiment, the height of the contact surface of the ophthalmic imaging device is automatically adjustable by a height adjustment mechanism, and the generated control signal is arranged to cause the height adjustment mechanism to automatically adjust the height of the contact surface to the target height.

In the first example aspect set out above, the value of first indicator is indicative of a height at which the eye of the subject was disposed when the one or more images were acquired, and the at least one mapping maps each value of the first indicator to a corresponding value of the second indicator such that a height of an imaging axis of the ophthalmic imaging device when the contact surface is disposed at the target height indicated by the value of the second indicator is smaller than the height indicated by the value of first indicator. The at least one mapping may be dependent on an age of the subject such that, for each value of the first indicator, the respective target height indicated by the corresponding value of the second indicator increases with increasing age of the subject, and the processor may be further arranged to receive an indication of the age of the subject, and to use the received indication of the age and the at least one mapping to map the value of the first indicator to the value of the second indicator. Additionally or alternatively, the at least one mapping may be dependent on a distance of the subject from the contact surface where the subject is in the position beside the ophthalmic imaging device such that, for each value of the first indicator, the respective target height indicated by the corresponding value of the second indicator decreases with increasing distance, and the processor may be further arranged to acquire an indication of the distance, and to use the acquired indication of the distance and the at least one mapping to map the value of the first indicator to the value of the second indicator. The processor may be arranged to acquire the indication of the distance by processing at least some of the one or more images acquired by the at least one camera. Alternatively, the control system may further comprise a distance sensor arranged to measure the distance of the subject from the contact surface where the subject is in the position beside the ophthalmic imaging device, and the processor may be arranged to acquire the indication of the distance by receiving the measured distance from the distance sensor.

The at least one camera may be arranged to acquire the one or more images when the subject is sitting in a seated position on a seat beside the ophthalmic imaging device, the at least one mapping may be dependent on a height of the seat such that, for each value of the first indicator, the respective target height indicated by the corresponding value of the second indicator increases with increasing height of the seat, and the processor may be further arranged to acquire an indication of the height of the seat, and to use the acquired indication of the height of the seat and the at least one mapping to map the value of the first indicator to the value of the second indicator.

In the first example aspect of any of its example embodiments or variants thereof set out above, the contact surface may be movable in a lateral direction towards the head of the subject, the control system may be further arranged to generate a second control signal for moving the contact surface along the lateral axis to a target lateral position at which the contact surface is to contact the head of the subject during the imaging of the eye of the subject by the ophthalmic imaging device, and the processor may be further arranged to: process the one or more images to generate a value of a third indicator which is indicative of a separation along the lateral axis between the contact surface and a first lateral position at which the at least a portion of the head is disposed when the subject is sitting in the seated position on the seat beside the ophthalmic imaging device; use at least one second mapping to map the value of the third indicator to a value of a fourth indicator which is indicative of the target lateral position at which the contact surface is to contact the head of the subject during the imaging of the eye of the subject by the ophthalmic imaging device; and generate a second control signal for moving the contact surface to the target lateral position indicated by the value of a fourth indicator. Alternatively, the contact surface may be movable in a lateral direction towards the head of the subject, the control system may be further arranged to generate a second control signal for moving the contact surface along the lateral axis to a target lateral position at which the contact surface is to contact the head of the subject during the imaging of the eye of the subject by the ophthalmic imaging device, the control system may further comprise a distance sensor arranged to generate a value of a third indicator which is indicative of a separation along the lateral axis between the contact surface and a first lateral position at which the at least a portion of the head is disposed when the subject is sitting in the seated position on the seat beside the ophthalmic imaging device, and the processor may be further arranged to: use at least one second mapping to map the value of the third indicator to a value of a fourth indicator which is indicative of a target lateral position at which the contact surface is to contact the head of the subject during the imaging of the eye of the subject by the ophthalmic imaging device; and generate a second control signal for moving the contact surface along the lateral axis to the target lateral position indicated by the value of a fourth indicator. In either case, the at least one second mapping may map values of the third indicator to corresponding values of the fourth indicator, the at least one second mapping may be dependent on an age of the subject such that, for each value of the first indicator, the respective target lateral position indicated by the corresponding value of the fourth indicator comes closer to the first lateral position with increasing age, and the processor may be further arranged to receive an indication of the age of the subject, and to use the received indication of the age of the subject and the at least one second mapping to map the value of the third indicator to the value of the fourth indicator.

There is provided, in accordance with a second example aspect herein, a system for imaging an eye of a subject. The system comprises an ophthalmic imaging device arranged to image the eye of the subject, the ophthalmic imaging device comprising a height-adjustable contact surface arranged to contact a head of the subject during the imaging of the eye. The system further comprises a movement mechanism, which is arranged to adjust a height at which the height-adjustable contact surface is disposed to a target height at which the contact surface is to contact the head of the subject during the imaging of the eye. The system further comprises the control system according to the first example aspect herein, which is arranged to generate a control signal for controlling the movement mechanism to adjust the height to the target height. The movement mechanism may comprise a height-adjustable table, which is arranged to support the ophthalmic imaging device.

The system may further comprise at least one sensor of an object, each being one of a distance sensor arranged to measure a distance to the object and a proximity sensor arranged to detect the object when the object is within a detection range of the proximity sensor, wherein the at least one sensor and the control system are arranged to determine whether at least one of the height-adjustable table or the ophthalmic imaging device has been moved to within a predetermined distance of the object and, in response to determining that the at least one of the height-adjustable table or the ophthalmic imaging device has been moved to within the predetermined distance of the object, generating an instruction to stop an adjustment of the height to the target height being made by the movement mechanism. The system may alternatively further comprise at least one distance sensor arranged to measure a distance to an object, wherein the at least one distance sensor and the control system are arranged to determine whether at least one of the height-adjustable table or the ophthalmic imaging device is to be moved to within a predetermined distance of the object during an adjustment of the height to the target height by the movement mechanism, and generate a warning for a user in response to determining that the at least one of the height-adjustable table or the ophthalmic imaging device is to be moved to within the predetermined distance of the object during the adjustment.

The system may additionally or alternatively further comprise at least one sensor of an object, each sensor being one of a distance sensor arranged to measure a distance to the object and a proximity sensor arranged to detect the object when the object is within a detection range of the proximity sensor, wherein the at least one sensor and the control system are arranged to determine whether the subject is within a predetermined distance of the ophthalmic imaging device and, in response to determining that the subject is within the predetermined distance of the ophthalmic imaging device, control the at least one camera of the control system to acquire the one or more images.

### [Brief Description of the Drawings]

Example embodiments will now be explained in detail, by way of non-limiting example only, with reference to the accompanying figures described below. Like reference numerals appearing in different ones of the figures can denote identical or functionally similar elements, unless indicated otherwise.
Figure 1 is a schematic illustration of system for imaging an eye of a subject according to an example embodiment herein.
Figure 2 is a schematic illustration of a control system according to the example embodiment herein.
Figure 3A is a schematic illustration of a subject seated beside a system for imaging an eye of the subject according to an example embodiment herein, before the chinrest of the ophthalmic imaging device has been moved to a target position suitable for the subject.
Figure 3B is a schematic illustration of the subject seated beside the system of the example embodiment, after the chinrest has been moved to the target position.
Figure 3C is a schematic illustration of the system of the example embodiment in the same state as in Figure 3B, and the subject before after moving to engage with the chinrest.
Figure 4 is a schematic illustration of a programmable signal processing hardware, which may be configured to perform the functions of the processor described herein.
Figure 5 is a flow diagram illustrating a process by which the processor of the control system generates a first control signal in the example embodiment herein.
Figure 6 is a schematic illustration of a first mapping described herein, in the example form of a table.
Figure 7 is a flow diagram illustrating a process by which the processor of the control system generates a second control signal in the example embodiment herein.
Figure 8 is a schematic illustration of a second mapping described herein, in the example form of a table.

### [Detailed Description of Example Embodiments]

To address the above-described problems, the present inventors have devised a control system in accordance with an example embodiment, which is arranged to generate a control signal for adjusting a height at which a height-adjustable contact surface of an ophthalmic imaging device is disposed to a target height at which the contact surface is to contact a head of a subject during imaging of an eye of the subject by the ophthalmic imaging device. The control system includes at least one camera, which is arranged to acquire one or more images of at least a portion of the head of the subject where the subject is in a position, beside the ophthalmic imaging device, for the head to be brought into contact with the contact surface when the contact surface is at the target height. The control system also has a processor, which is arranged to process the one or more images to generate a value of a first indicator, which is indicative of a height at which the at least a portion of the head was disposed when the one or more images were acquired. Instead of automating the conventional approach to making patient interface height adjustments, where the difference in height between the patient interface and the head is judged by the operator and minimised by the operator iteratively adjusting the height of the patient interface and evaluating the height difference resulting from the previous adjustment, the processor of the example embodiment may acquire the target height, at which the contact surface is to contact the head during the imaging, in a faster, non-iterative way, by using a mapping M to map the value of the first indicator to a value of a second indicator, which is indicative of the target height of the contact surface, and generating a control signal for adjusting the height at which the contact surface is disposed to the target height indicated by the value of the second indicator. The mapping M may, for example, be provided in the form of a look-up table (LUT), which associates values of the first indicator with corresponding values of the second indicator, and the processor may use the value of the first indicator to look up the corresponding value of the second indicator, which is indicative of the target height of the contact surface, in the LUT.

In some example embodiments, the mapping may be dependent on how far the subject is seated or stood from the ophthalmic imaging device (which may be measured, estimated or assumed), in such a way that the adjustment of the height of the contact surface to the target height indicated by the value of the second indicator allows the subject to lean forward and engage with the contact surface whilst remaining comfortable, which may help the subject to maintain the position of the eye more steadily during imaging of the eye by the ophthalmic imaging device and thereby reduce motion artifacts in the acquired image(s). The mapping may be additionally or alternatively depend on the age of the subject, and reflect the observation that elderly subjects have lower mobility than younger subjects and, in particular, tend to have a very limited ability adjust their eye height once settled into a particular seated posture as compared to younger subjects when engaging with the ophthalmic imaging device's contact surface. The use of such a mapping to set the height of the ophthalmic imaging device's contact surface may therefore allow subjects, and elderly subject in particular, to maintain the position of the eye more steadily during imaging and thereby reduce motion artifacts in the acquired image.

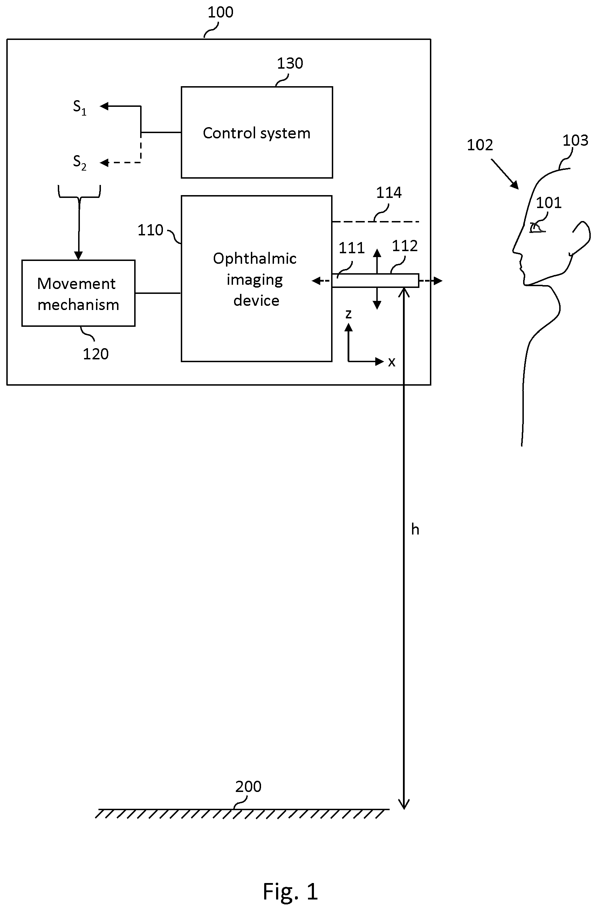

Figure 1 is a schematic illustration of a system 100 for imaging an eye 101 of a (human) subject 102. The system 100 comprises an ophthalmic imaging device 110, a movement mechanism 120 and a control system (also referred to as a guidance system) 130.

The ophthalmic imaging device 110 is arranged to image the eye 101 of the subject 102. The ophthalmic imaging device 110 may, as in the present example embodiment, be an optical coherence tomography (OCT) imaging device in the form of a swept-source OCT (SS-OCT) imaging device. The ophthalmic imaging device 110 may, however, be another kind of Fourier-domain OCT (FD-OCT) imaging device, such as a spectral-domain OCT (SD-OCT) imaging device, or may alternatively be a time-domain OCT (TD-OCT) imaging device. However, the ophthalmic imaging device 110 is not so limited and may be any other type of ophthalmic imaging device for imaging the posterior segment of the eye 101, such as a scanning laser ophthalmoscope (SLO) or a fundus camera, for example. Furthermore, the ophthalmic imaging device 110 need not be limited to imaging the posterior of the eye 101 and may alternatively or additionally be arranged to image the anterior segment of the eye 101.

The ophthalmic imaging device 110 may include well-known components, such as a light beam generator, a scanning system, an interferometer, a light detector, OCT data processing hardware, and a scan head (not shown). The scanning system may be arranged to perform a one- and/or two-dimensional point-scan of a light beam across the retina of the eye 101 and collect light which has been scattered by the retina during the point scan. The ophthalmic imaging device 110 may thus acquire A-scans at respective scan locations that are distributed across a surface of the retina, by sequentially illuminating the scan locations with the light beam, one scan location at a time, and collecting at least some of the light scattered by the retina at each scan location. The ophthalmic imaging device 110 may be arranged to acquire OCT images in the form of B-scans by performing the point-scan to acquire successive A-scans along, for example, a straight line. However, the ophthalmic imaging device 110 may alternatively be arranged to acquire the B-scans by the scanning system performing line-scans, using hardware well-known to those versed in the art. More generally, the ophthalmic imaging device 110 may be arranged to acquire OCT images in the form of B-scans or C-scans by performing point-scans or a line-scans using predetermined scanning patterns well-known to those versed in the art (e.g. a spiral scan), or by employing a full-field set-up.

The ophthalmic imaging device 110 comprises a height-adjustable patient interface 111 having a contact surface 112, which surface is arranged to contact the head 103 of the subject 102 and help keep the head 103 steady during imaging of the eye 101. A patient interface 111 serving this purpose may take one of many different forms.

For example, the patient interface 111 may, as in the present example embodiment, be provided in the form of a chinrest, which has an upward-facing contact surface 112 on which the chin of the subject 102 rests during imaging of the eye 101 by the ophthalmic imaging device 110.

As another example, the patient interface 111 may be provided in the form of a forehead rest (which may also be referred to as a headrest). In some example embodiments, the forehead rest may be arranged to contact only the forehead of the subject 102 when the subject 102 engages with the forehead rest. In other example embodiments, the forehead rest may be shaped not only to contact the subject's forehead but also a portion of the subject's face surrounding the eye sockets when the subject 102 engages with the forehead rest, thereby helping to suppress lateral movements of the head 103, as well as movements of the head 103 forwards and backwards, when the subject's head 103 is engaged with the forehead rest. In such other example embodiments, the forehead rest may be shaped to fit around the eye sockets and over the nasal bridge of the subject so as to provide a similarly shaped contact surface on the subject's head 103 as that of a ski mask or snorkelling mask, for example.

As some further examples, the patient interface 111 may be provided in the form of a combination of the chinrest and the forehead rest as described above, or in the form of one or two eyecups (among other possibilities).

The height of the contact surface 112, h, may be adjustable relative to some of the remaining components of the ophthalmic imaging device 110. However, in the present example embodiment, the height h of the contact surface 112 relative to the remainder of the ophthalmic imaging device 110 is fixed, and is adjustable by vertical movement of the ophthalmic imaging device 110 as a whole, specifically by an upward and downward movement (along the z-axis in Figure 1) of a top surface of a height-adjustable table, on which the ophthalmic imaging device 110 rests. The height h is measured relative to a reference height, such as ground level 200, which may be the floor of a room containing the ophthalmic imaging device 110. The contact surface 112 may, as in the present example embodiment, also be movable horizontally towards and away from the subject 102 (along the x-axis in Figure 1) so as to change its lateral position, as described in detail below, although such horizontal adjustability of the contact surface 112 may not be available in some example embodiments.

The movement mechanism 120 is arranged to adjust the height h, at which the height-adjustable contact surface 112 is disposed, to a target height at which the contact surface 112 is to contact the head 103 of the subject 102 during the imaging of their eye 101. The movement mechanism 120 may, as in the present example embodiment, be further arranged to move the contact surface 112 horizontally to a target lateral position, at which the contact surface 112 is to contact the head 103 of the subject 102 during the imaging.

The movement mechanism 120 may, as in the present example embodiment, comprise a height-adjustable table, which is arranged to support the ophthalmic imaging device 110 on a top surface of the table. The ophthalmic imaging device 110 thus rests on top of the height-adjustable table. The height of the top surface of the height-adjustable table is thus adjustable and may, as in the present example embodiment, also be movable horizontally towards and away from the head 103 of the subject 102. Thus, in example embodiments like the present, where the contact surface 112 is fixed relative to the rest of the ophthalmic imaging device 110, a vertical adjustment to change the height of the top surface of the table and a horizontal adjustment to change its lateral position results in a corresponding adjustment of the height of the contact surface 112 and its lateral position, respectively. Any suitable height-adjustable table may be used to support the ophthalmic imaging device 110 and optionally allow it to move horizontally towards and away from the subject 102 (e.g. the Optos^{®} Table). The height-adjustable table may have a motorised movement mechanism which is controllable by the operator (e.g. via buttons on the table or a handset, that can be pressed by the operator) to adjust the height of the top surface of the table and optionally also its lateral position.

However, the movement mechanism 120 may be provided in other forms, for example a height-adjustable wall mount (e.g. a height-adjustable wall-mounted arm), which is attached to a wall of the room and is arranged to support the ophthalmic imaging device 110 on a height-adjustable top surface of the wall mount. The top surface of the height-adjustable wall mount, which supports the ophthalmic imaging device 110, may also be movable horizontally towards and away from the head 103 of the subject 102. Thus, where the contact surface 112 is fixed relative to the rest of the ophthalmic imaging device 110, a vertical adjustment to change the height of the top surface of the wall mount and a horizontal adjustment to change its lateral position results in a corresponding adjustment of the height of the contact surface 112 and its lateral position, respectively. The height-adjustable wall mount may comprise a motorised movement mechanism which is controllable by the operator (e.g. via buttons on the wall mount or a handset, that can be pressed by the operator) to adjust the height of the top surface of the wall mount and optionally also its lateral position.

Although the adjustment of the height and, optionally, lateral position of the contact surface 112 is described above as being achieved by movement of the ophthalmic imaging device 110 as a whole by a movement mechanism 120 which supports the ophthalmic imaging device 110, these adjustments of the contact surface 112 may be performed in other ways. For example, the position of the contact surface 112 may be fixed in relation to a portion of the ophthalmic imaging device 110 comprising the scan head and the scanning system, and the portion may be arranged to have an adjustable height relative to the remaining components of the ophthalmic imaging device 110, including the interferometer, the detector, the light source and the OCT data processing hardware. Such an arrangement may be achieved by optically coupling the scanning system to the interferometer with an optical fibre, for example, and providing a mechanism employing a stepper motor or the like to move the scanning system, the scan head and the patient interface 111 comprising the contact surface 112 relative to the remaining components of the ophthalmic imaging device 110.

The control system 130 is arranged to generate a first control signal S₁ for adjusting the height h to the target height. The control system 130 may, as in the present example embodiment, be further arranged to generate a second control signal S₂ for moving the contact surface 112 horizontally, to change its lateral position to a target lateral position, at which the contact surface 112 is to contact the head 103 of the subject 102 during the imaging of the eye 101 by the ophthalmic imaging device 110. However, the generation of the second control signal S₂ by the control system 130 is optional and may be omitted in some example embodiments.

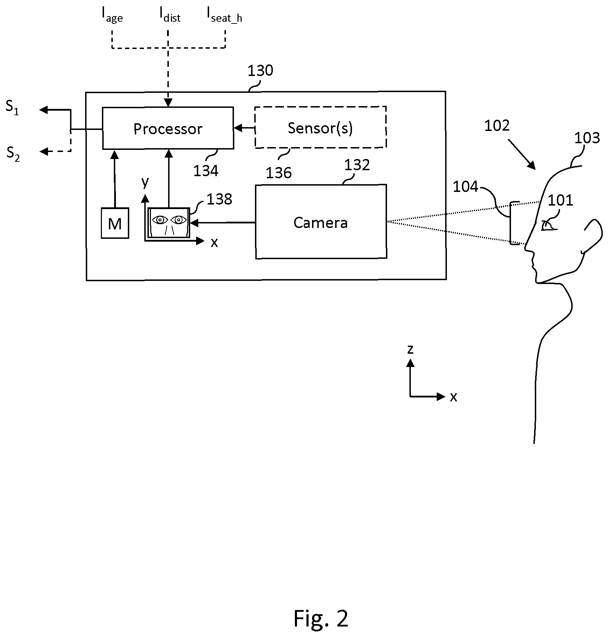

Figure 2 is a schematic illustration showing details of the control system 130. The control system 130 comprises at least one camera 132, at least one processor 134 and may further comprise one or more sensors 136, which are described in more detail below.

In the present example embodiment, the control system 130 comprises a single camera 132, which is arranged to acquire an image 138 of at least a portion 104 of the head 103 of the subject 102, where the subject 102 is in a body position beside the ophthalmic imaging device 110 suitable for the head 103 to be brought into contact with the contact surface 112 when the contact surface 112 is at the target height, for example by the subject 102 leaning forward to engage with the patient interface 111 such that the head 103 comes into contact with the contact surface 112. The camera 132 may be the camera of an automatic pupil alignment module (also known as a patient alignment module, PAM) of the ophthalmic imaging device 110, which serves to automatically align the imaging beam of the imaging device 110 with the pupil during micro-alignment, or a dedicated digital camera for the control system 130. The subject 102 may, for example, be in a seated position on a seat beside the ophthalmic imaging device 110, from which position the subject 102 can move their head 103 forward by leaning towards the ophthalmic imaging device 110 to contact the contact surface 112 with their head 103 after the contact surface 112 has been moved to the target height. Alternatively, and typically where the subject 102 is a child, the subject 102 may be stood (i.e. be in standing position) beside the ophthalmic imaging device 110, from which the subject 102 can move their head 103 forward by leaning towards the ophthalmic imaging device 110 to contact the contact surface 112 with their head 103 after the contact surface 112 has been moved to the target height. The imaged portion 104 of the head 103 may be the portion of the head 103 which is within the field-of-view (FoV) of the camera 132 (or within a combined FoV of two or more cameras, in example embodiments when more than one camera 132 is present) when the subject 102 is in the body position beside the ophthalmic imaging device 110 for their head 103 to be brought into contact with the contact surface 112 once the contact surface 112 has been set at the target height.

Figures 3A to 3C are schematic illustrations of a subject 102 in a seated position beside the ophthalmic imaging device 110 of the system 100, at different stages of the macro alignment process, which will now be described. As noted above, the movement mechanism 120 of the system 100 is provided in the example form of a height-adjustable table in the present example embodiment, which is shown at 300 in Figures 3A to 3C. Components of the control system 130, including the camera 132, the processor 134 and sensors 136-1, 136-2 and 136-3 (as examples of the one or more sensors 136 in Figure 2) are also shown in Figures 3A to 3C. These figures also schematically illustrate a user interface 113, which the processor 134 is arranged to control in order to guide the operator (user) of the ophthalmic imaging device 110 to set the height of the contact surface 112 to the target height, and the lateral position of the contact surface 112 to the target lateral position for the imaging.

As shown in Figure 3A, the subject 102 is initially sat in a seated position (posture) on a seat 400 beside the ophthalmic imaging device 110, ready to have their eye 101 imaged while remaining seated on the seat 400. Although the seat 400 is provided in the form of a chair in the present example embodiment, it may be the subject's wheelchair, for example, in other example embodiments. With the subject 102 thus seated, the camera 132 acquires an image 138 of the head 103 of the subject 102, for example in response to a start command is input to the control system 130 by the operator, or automatically in response to the detection of the presence of the subject 102, as described below. The camera 132 is arranged such that its field-of-view captures the head 103 of the subject 102 whilst preferably avoiding any part of the ophthalmic imaging device 110, such as the patient interface 111.

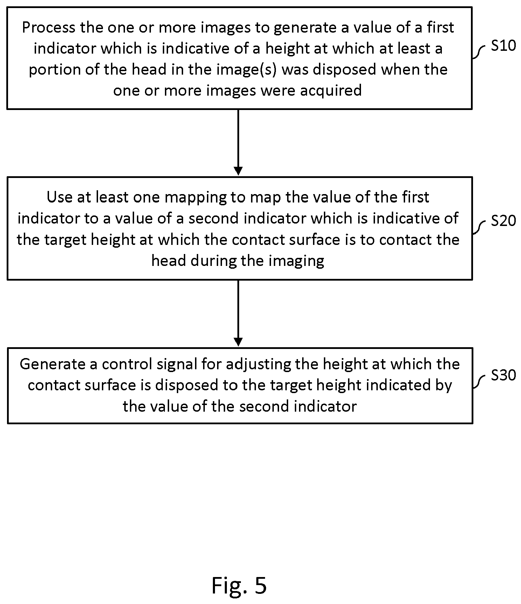

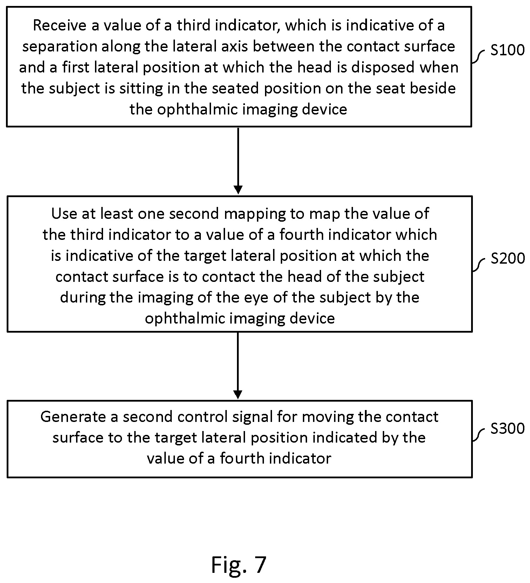

The processor 134 is arranged generate the first control signal S₁ and, optionally, the second control signal S₂, based on the acquired image 138. Processes by which the processor 134 may generate the first control signal S₁ and the second control signal S₂ are described in detail below, with reference to Figures 5 and 7, respectively.

The height h of the contact surface 112 and its lateral position may, as in the present example embodiment, be adjustable by the operator, using any well-known kind of mechanical or operator-controlled electrical drive mechanism for moving the top of the height-adjustable table 300 vertically (along the z-axis) and horizontally towards and away from the subject 102 (i.e. along the x-axis). In either case, the user interface 113 (which may comprise a screen viewable by the operator) is controlled by the processor 134, using the first control signal S₁ and the second control signal S₂, to provide the operator with instructions, for example in the form of "up", "down", "forward" and "backward" direction indicators (e.g. in the form of arrows or triangles), for setting the height and the lateral position of the contact surface 112 to the target height and the target lateral position, respectively. In some example embodiments, the user interface 113 may comprise a display of the ophthalmic imaging device 110, which is used to control the ophthalmic imaging device 110 and view acquired images. The user interface 113 may additionally or alternatively comprise a speaker for providing the user with audio instructions for adjusting the height h and lateral position d of the contact surface 112.

The control system 130 may alternatively automatically control one or more electrical actuators (e.g. electrical motors) that may be included in the height-adjustable table 300, using the first control signal S₁ and the second control signal S₂, to adjust the height h, at which the height-adjustable contact surface 112 is disposed, from an initial height h₀ to the target height h_{T} at which the contact surface 112 is to contact the head 103 of the subject 102 during the imaging of the eye 101, and to adjust the lateral position of the contact surface 112 (which may be expressed as a distance d of a point on the contact surface 112 from a stationary reference point on the height-adjustable table 300, in a direction along the x-axis towards the subject 102) from an initial position at distance d₀ to the target lateral position at distance d_{T}. The control system 130 may thus guide the patient interface 111 to a position that is suitable for the subject 102 to engage with it comfortably.

Figure 3B shows the result of the operator adjusting the height and lateral position of the top of the height-adjustable table 300, in accordance with instructions provided by the control system 130 via a display and/or speaker of the user interface 113, to correspondingly adjust the height and lateral position of the contact surface 112 to the target height h_{T} and the target lateral position at distance d_{T}, respectively. The user interface 113 may indicate to the operator when the target height and lateral position of the contact surface 112 have been reached, based on vertical and horizontal movements of the top of the height-adjustable table 300 that may be monitored by the control system 130. The subject 102 may be directed to maintain the seated position while the movement of the height-adjustable table 300 occurs to reduce the risk of the height-adjustable table 300 or the ophthalmic imaging device 110 thereon colliding with the subject 102 during the macro-alignment.

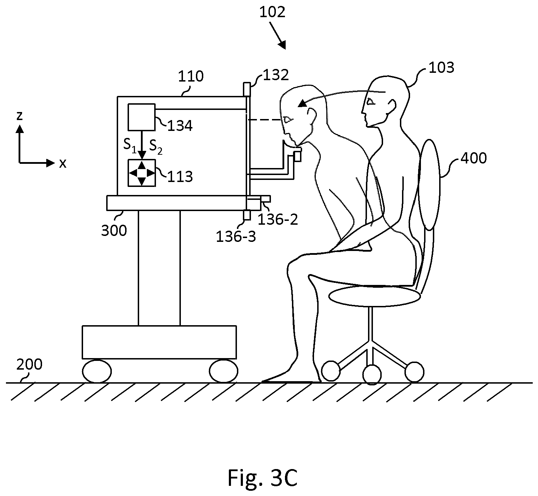

When the contact surface 112 has been moved to the target height and lateral position during the macro-alignment then, as shown in Figure 3C, the subject 102 registers with the patient interface 111 by leaning forward to engage with the patient interface 111 (chinrest in the present example embodiment), such that their chin contacts the contact surface 112 on the chinrest. The subject 102 maintains the resulting seated position until the imaging of the eye 101 by the ophthalmic imaging device 110 (and any prior micro-alignment that may be performed by the ophthalmic imaging device 110 to bring the eye 101 (e.g. the pupil centre) in closer alignment with the imaging axis 114) has been completed. During imaging of the eye 101 by the ophthalmic imaging device 110, there is no further movement of the height-adjustable table 300, and the subject 102 remains as still as possible. The hips of the subject 102 typically remain in substantially the same place while the subject 102 leans forward, with the seat 400 also remaining in place. Alternatively, the seat 400 may be moved forward by the subject 102, as described further below.

Although the subject 102 adopts a seated position beside the ophthalmic imaging device 110, from which their head 103 can be brought into contact with the contact surface 112 by the subject 102 leaning forward to engage with the patient interface 111 once the contact surface 112 has been adjusted to be at the target height h_{T}, the subject 102 need not be seated and may alternatively remain in a standing position during the macro-alignment process and subsequent micro-alignment (if any) and imaging by the ophthalmic imaging device 110. For example, where the ophthalmic imaging device 110 is used to image the eye of a child, then the ophthalmic imaging device 110 may be used where the subject 102 adopts a standing position besides the ophthalmic imaging device 110, which is suitable for the head 103 to be brought into contact with the contact surface 112 when the contact surface 112 is at the target height. Accordingly, when the contact surface 112 has been set at the target height, the standing subject 102 may lean forward and contact the contact surface 112 for the duration of the imaging of the eye 101 by the ophthalmic imaging device 110.

Referring again to Figure 2, the processor 134 may, as in the present example embodiment, be further arranged to receive an indication, I_{age}, of the age of the subject 102, the use of which is described below. The indicator I_{age} may be input to the processor 134 by the operator or may be received from an external computer (e.g. PC or server), as part of the subject's patient record, for example.

The processor 134 may, as in the present example embodiment, be further arranged to acquire an indication I_{dist} of a distance of the subject 102 from the contact surface 112 while the subject 102 is in the aforementioned position beside the ophthalmic imaging device 110. For example, the processor 134 may be arranged to acquire the indication of the distance I_{dist} by processing at least some of the one or more images acquired by the at least one camera 134. Where the control system 130 comprises a single camera 132, the processor 134 may use known object detection techniques based on machine learning, such as a Haar Cascade classifier, to identify the pupils of the subject's eyes in an image acquired by the camera 132, use the identified pupil locations to determine the interpupillary distance in the image (in pixels), and then convert the determined interpupillary distance in pixels to an estimate for the distance of the subjects' eyes from the contact surface 112. This conversion may be performed using a mapping in the form of a conversion function or look-up table, for example, obtained by measuring the respective distance (in pixels) in each image of a set of images captured by the camera 132 of a calibration board showing two markers that are spaced apart by about 63 mm (the average interpupillary distance in adults) which is disposed at different known distances from the contact surface 112 in the set of images. The estimate may be improved using a measured interpupillary distance of the subject 102 if input by the operator or otherwise made available to the processor 134 (e.g. from a patient record of the subject 102 retrieved from a remote data store). In other example embodiments, where the control system 130 comprises a stereo vision system having two cameras with parallel optical axes that are separated from one another, which is arranged to acquire stereoscopic images of the head 103 of the subject 102, the processor 134 may estimate the distance of the subject 102 from the cameras (hence from the contact surface 112) by processing the images using well-known techniques for distance estimation from stereo vision.

As a further alternative, the control system 130 may, as in the present example embodiment, further comprise a distance sensor as one of the sensors 136, which is arranged to measure the distance of the head 103 or trunk of the subject 102 from the contact surface 112 (e.g. a reference point on the contact surface, such as the closest point on the contact surface to the subject 102) when the subject 102 is in the aforementioned position beside the ophthalmic imaging device 110. The processor 134 may be arranged to acquire the indication I_{dist} by receiving the measured distance from a distance sensor as shown at 136-1 in Figures 3A to 3C, which is described in more detail below. The distance sensor 136-1 may be of any known kind, such as an ultrasonic sensor, an infrared (IR) distance sensor, or a light detection and ranging (LIDAR) sensor, for example. The distance sensor 136-1 may, as in the present example embodiment, be located beside the patient interface 111 so as to measure the distance d_{DS} between the distance sensor 136-1, and thus equivalently between the contact surface 112 of the patient interface 111, and the head 103 or trunk of the subject 102. In this case, the processor 134 may be arranged to acquire the indication I_{dist} of the distance d_{DS} by receiving the measured distance from the distance sensor 136-1. However, the distance sensor 136-1 may be located elsewhere on the ophthalmic imaging device 110, and the processor 134 may be arranged to correct the distance measured by the distance sensor 136-1 to determine the distance d_{DS} using the distance along the x-axis between the distance sensor 136-1 and the contact surface 112 (e.g. the aforementioned reference point on the contact surface 112).

Referring again to Figure 2, the processor 134 may, as in the present example embodiment, be further arranged to acquire an indication, I_{seat_h}, of the height of the seat 400, the use of which is described below. This indication I_{seat_h} may be input to the processor 134 by the operator, or may be acquired from a seat height sensor of the control system 130 (not shown), which is arranged to measure the height of the seat 400 from ground level 200 and transmit this to the processor 134.

The one more sensors 136 of the control system 130 shown in Figure 2 may, as in the present example embodiment, comprise at least one sensor of an object, each being a distance sensor which is arranged to measure a distance to the object, or a proximity sensor which is arranged to detect the object when the object is within a detection range of the proximity sensor. The distance sensor may be of any known kind, such as an ultrasonic sensor, an infrared (IR) distance sensor, or a light detection and ranging (LIDAR) sensor, for example. The distance sensor may alternatively comprise a digital camera, which is arranged to capture an image which includes the object as well as another object, and a processor arranged to process the image to estimate the distance between the objects, using any known technique. The proximity sensor may be of any known kind, for example a contact switch or a contactless proximity sensor such an IR or ultrasonic proximity sensor, for example.

Three such object sensors are provided in the present example embodiment, namely the distance sensor 136-1 described above, as well as proximity sensors 136-2 and 136-3 shown in Figures 3A to 3C. Each object sensor and the processor 134 of the control system 130 may, as in the present example embodiment, be arranged to determine whether one or both of the height-adjustable table 300 or the ophthalmic imaging device 110 has been moved to within a predetermined distance of the object (e.g. a part (e.g. leg) of the subject 102 or, when the subject 102 is sitting on a seat 400, a part (e.g. armrest) of the seat 400). The distance sensor 136-1 is used by the processor 134 to determine whether a part of the ophthalmic imaging device 110 closest to the subject 102 (e.g. the patient interface 112) has been moved to within a first predetermined distance of subject 102 (by comparing distance values measured by the distance sensor 136-1 with a first predetermined threshold), the proximity sensor 136-2 is arranged to sense whether the height-adjustable table 300 has been moved to within a second predetermined distance of the subject 102 or the seat 400, and the proximity sensor 136-3 is arranged to sense whether the height-adjustable table 300 has been moved to within a third predetermined distance of the subject 102 or the seat 400. The first, second and third predetermined distances may be different from one another, although two or more of these predetermined distances may be the same. Each of the proximity sensors 136-2 and 136-3 is further arranged to transmit to the processor 134 a respective signal in response to sensing that the height-adjustable table 300 or the ophthalmic imaging device 110 (as the case may be) has been moved to within the respective predetermined distance of the subject 102 or the seat 400 (as the case may be).

Similarly, the processor 134 compares distance values reported by the distance sensor 136-1 with the first predetermined threshold, and generates an instruction to stop the adjustment of the height of the height-adjustable table 300 when the reported distance becomes smaller than the first threshold value.

In response to determining that at least one of the height-adjustable table 300 and the ophthalmic imaging device 110 (as the case may be) has been moved to within the predetermined distance of the object based on the signals received from the object sensors, the processor 134 may generate an instruction to stop an adjustment of the height to the target height being made by the movement mechanism 120. For example, the processor 134 may compare distance values reported by the distance sensor 136-1 with the first predetermined threshold, and generate the instruction when the reported distance becomes smaller than the first threshold value. The instruction may be displayed on the user interface 113 where the operator adjusts the height of the height-adjustable table 300 by operating a mechanical drive mechanism or controlling an electromechanical drive mechanism. Alternatively, the control system 130 may use the instruction to automatically control one or more electrical actuators (e.g. electrical motors) that may be included in the height-adjustable table 300.

In addition or as an alternative to its use in collision avoidance as described above, the distance sensor 136-1 may be used to initiate imaging of the subject's head 103 by the camera 132. More particularly, the processor 134 of the control system 130 may be arranged to determine whether the subject 102 is within a predetermined distance of the ophthalmic imaging device 110 and, in response to determining that the subject 102 is within the predetermined distance of the ophthalmic imaging device 110, control the camera 132 to acquire the image 138. The imaging of the subject's head 103 by the camera 132 may alternatively be initiated by a proximity sensor, which is arranged to trigger the imaging once it has detected the subject 102 within its detection range.

As an alternative to the reactive approach to avoiding collisions of at least one of the height-adjustable table 300 and the ophthalmic imaging device 110 with the object (e.g. a part of the subject 102 or a part of the seat 400), a predictive approach to collision avoidance may instead be taken. In this case, at least one distance sensor may be provided, such as distance sensor 136-1, which is arranged to measure a distance to an object such the subject 102, wherein the distance sensor and the control system 130 are arranged to determine whether at least one of the height-adjustable table 300 or the ophthalmic imaging device 110 is to be moved to within a predetermined distance of the object during an adjustment of the height h of the contact surface 112 to the target height h_{T} by the movement mechanism 120, and generate a warning for the operator in response to determining that the at least one of the height-adjustable table 300 or the ophthalmic imaging device 110 is to be moved to within the predetermined distance of the object during the adjustment. The warning may be conveyed to the operator via the one or both of the display and the speaker of the above-described user interface 113, for example.

Although the one or more sensors 136 of Figure 2 have been exemplified by the sensors 136-1, 136-2 and 136-2 shown in Figures 3A to 3C, the present disclosure is not so limited to these examples, and any number of sensors of any of the kinds described above or similar kinds may be used. In particular, multi-directional distance sensors may be used to reduce the total number of required sensors.

The processor 134 may be provided in any suitable form, for example as a processor 520 of a programmable signal processing hardware 500 of the kind illustrated schematically in Figure 4. The components of the programmable signal processing hardware 500 may be included within the control system 130. The programmable signal processing apparatus 500 comprises a communication interface (I/F) 510, for receiving the image 138 from the camera 132 (or multiple images from multiple cameras in other example embodiments) and, optionally, at least one of the indications I_{age}, I_{dist} and I_{seat_h} described above and, where provided, the second control signal S₂, to the user interface 113 or to the movement mechanism 120 for automatic adjustment of the height and lateral position of the contact surface 112, as described above. The signal processing hardware 500 further comprises a processor 520 (e.g. a Central Processing Unit, CPU, and/or a Graphics Processing Unit, GPU), a working memory 530 (e.g. a random-access memory) and an instruction store 540 storing a computer program 545 comprising the computer-readable instructions which, when executed by the processor 520, cause the processor 520 to perform various functions of the processor 134 described herein.

The working memory 530 stores information used by the processor 520 during execution of the computer program 545, including a mapping M for mapping the value of the first indicator, which is indicative of a height at which the head 103 (or an anatomical feature thereof) was disposed when the image 138 was acquired, to a corresponding value of the second indicator, which is indicative of the target height h_{T} of the contact surface 112. The working memory 530 may also store a further mapping described below for converting a y-axis component of a pixel location of a pixel in the image 138 to a height of an anatomical feature represented by the pixel. The working memory 530 may alternatively store a mapping in place of the aforementioned mappings, which mapping can be used to map a y-axis component of a pixel location of a pixel in the image 138 representing an anatomical feature such as the eye 101 directly to a corresponding value of the second indicator which is indicative of the target height h_{T} of the contact surface 112.

The instruction store 540 may comprise a ROM (e.g. in the form of an electrically erasable programmable read-only memory (EEPROM) or flash memory) which is pre-loaded with the computer-readable instructions. Alternatively, the instruction store 540 may comprise a RAM or similar type of memory, and the computer-readable instructions of the computer program 545 can be input thereto from a computer program product, such as a non-transitory, computer-readable storage medium 550 in the form of a CD-ROM, DVDROM, etc. or a computer-readable signal 560 carrying the computer-readable instructions. In any case, the computer program 545, when executed by the processor 520, causes the processor 520 to perform the functions of the processor 134 as described herein. In other words, the processor 134 of the present example embodiment may comprise a computer processor 520 and a memory 540 storing computer-readable instructions which, when executed by the computer processor 520, cause the computer processor 520 to perform the functions of the processor 134 as described herein.

It should be noted, however, that the processor 134 may alternatively be implemented in non-programmable hardware, such as an ASIC, an FPGA or other integrated circuit dedicated to performing the functions of the processor 134 described herein, or a combination of such non-programmable hardware and programmable hardware as described above with reference to Figure 4. Further, in some example embodiments, the programmable signal processing hardware 500 may further perform at least one of the functions of the OCT data processing hardware, where the ophthalmic imaging device 110 is an OCT imaging device, or functions of a controller of the movement mechanism 120, where provided.

Figure 5 is a flow diagram illustrating a process by which the processor 134 generates the first control signal S₁ for adjusting the height h at which the contact surface 112 is disposed to the target height, h_{T}. Note that the processor 134 may first receive the image 138 and any additional images of the head 103 (or portion thereof) from the camera 132 via the I/F 510, for example.

In process S10 of Figure 5, the processor 134 processes the one or more images acquired by the camera(s) of the control system 130 to generate a value of a first indicator which is indicative of a height at which the head 103 (or potion thereof) in the image(s) was disposed when the one or more images were acquired. The processor 134 may, as in the present example embodiment, generate the value of the first indicator by firstly identifying the eye 101 of the subject 102, although another anatomical feature of the head 103 may be identified in other example embodiments, for example the brow, the mouth, an ear or the chin of the subject 102. This may be achieved by use of an object detection algorithm of one of the kinds well-known to those skilled in the art. For example, the processor 134 may use known object detection techniques based on machine learning, such as a Haar Cascade classifier, to identify the eyes in the image 138 acquired by the camera 132, thus yielding pixel coordinates of the eye 101 in the image 138.

The y-axis component of the pixel coordinates is converted to a height of the eye 101 (e.g. relative to the camera 132 or ground level 200) using a mapping, which may be obtained by using the camera 132 to acquire a calibration image of a calibration board having a series of markers that are spaced vertically (for example, at regular intervals of e.g. 1 cm), with the board being located at a distance from the camera 132 that is equal to a typical distance at which the head 103 would be disposed when the subject 102 is in place, ready to have their eye 101 imaged. The y-axis component of each pixel location in the calibration image corresponding to a respective one of the markers on the calibration board may then be correlated with the height (relative to the (usually horizontal) optical axis of the camera 132 or ground level 200, for example) of the respective marker on the calibration board. Where, as in the present example embodiment, the control system 130 includes a distance sensor 136-1 to measure the distance d_{DS} between it (and thus equivalently between the contact surface 112) and the head 103 or trunk of the subject 102, a set of such calibration images of the calibration board at different distances along the x-axis from the distance sensor/patient interface 111 may be captured by the camera 132 to obtain a set of mappings of the kind described above, and the most appropriate mapping from among those mappings, which is to be used to convert the y-axis component of a pixel location of a pixel in the image 138 to a relative height of the portion of the subject's face represented by the pixel, may be selected in dependence on the distance d_{DS} measured by the distance sensor 136-1 during the macro-alignment process described above.

It is noted that the value of the first indicator may generally indicate the height of a single anatomical feature (e.g. mouth or chin) obtained as described above, or a mean height of two anatomical features (e.g. eyes or ears) within the image 138. Furthermore, the height of an anatomical feature of the head 103 (which may be taken to provide a measure of the height of the head 103) determined as described above may be used to estimate the height of another anatomical feature on the subject's face, using an average height difference between the features which may be derived from a sample of the population. This estimate may be useful to make where the height of the anatomical feature of interest, for example the chin, is more difficult to determine from the image 138 than another feature, for example the eye 101, which may have greater contrast in the image 138.

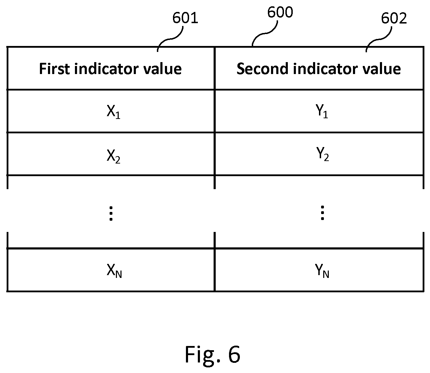

In process S20 of Figure 5, the processor 134 uses at least one mapping M to map the value of the first indicator to a value of a second indicator, which is indicative of the target height h_{T} at which the contact surface 112 is to contact the head 103 during the imaging of the eye 101. Figure 6 is a schematic illustration of an example mapping M in the form of a table 600. The table 600 comprises a first column 601 with values X₁, X₂, ... X_{N} of the first indicator and a second column 602 with corresponding values Y₁, Y₂, ... Y_{N} of the second indicator. However, the form of the mapping M is not so limited, and it may alternatively be provided as a function of multiple variables or a multi-dimensional look-up table, for example, as described in more detail below.

The mapping M maps each value Xᵢ of the first indicator to a corresponding value Yᵢ of the second indicator such that a height of an imaging axis 114 of the ophthalmic imaging device 110, when the contact surface 112 is disposed at the target height h_{T} indicated by the value of the second indicator, is smaller than the height of the eye 101 indicated by the value of first indicator. For example, the respective value Yᵢ of the second indicator corresponding to each value Xᵢ of the first indicator may indicate a target height h_{T} of the ophthalmic imaging device's chinrest which is such that, when the subject has leaned forward and placed their chin on the chinrest set at the target height h_{T}, and thereby reduced the height of their eye relative to the heigh indicated by the value Xᵢ of the first indicator, the subject's eye is approximately in line with the imaging axis 114 (in other words, at a height suitable for an image of the eye 101 to be acquired by the ophthalmic imaging device 110 or a height suitable for micro-alignment of the ophthalmic imaging device 110 with the eye 101 to be performed, after which the eye 101 can be imaged). Such mapping may be based on an average height difference between the eyes and the chin which is derived from a sample of the population, for example. The height of the imaging axis 114 above the contact surface 112 may likewise be set to correspond to such average height difference, for example. The difference between the height indicated by the value Xᵢ of the first indicator and the height of the subject's eye (or the imaging axis 114) when the subject's chin is resting on the chinrest at the target height h_{T} may be set to a predetermined value, which may be within the range of 2-10 cm, preferably 3-8 cm, and more preferably 5 cm, to reflect the natural downward motion of the head 103 shown in Figure 3C as the subject (assumed to be of an average height and mobility in this case) leans forward in a way and to a degree which they find comfortable, i.e. without straining to place their head in a position other than that which would follow from a relaxed leaning forward movement to a pose that they can comfortably maintain for the duration of the imaging session.

The extent to which the subject can lean forward to adopt a pose which they can comfortably maintain for the duration of the imaging session may depend on the age of the subject, as older subjects (for example, those beyond 50 years of age) tend to have more limited mobility than younger subjects, and are thus often able to lean forward less far whilst remaining comfortable. More particularly, the degree of hip flexion tends to decrease with increasing age past middle age, and this can reduce the extent to which the person is able to lean forward and comfortably maintain the resulting position for the duration of the imaging of their eye, and can consequently also reduce the amount by which the height of the head decreases as the subject leans forward.

To account for the consequent variability of the ideal target height h_{T} for the contact surface 112 amongst subjects who are of the same or similar height but different ages, the mapping M may, as in the present example embodiment, be dependent on the age of the subject 102 such that, for each value of the first indicator, the respective target height h_{T} indicated by the corresponding value of the second indicator increases with increasing age of the subject 102, at least where the age falls with a predetermined range, e.g. 50 years and above. This dependency of the mapping M on the age of the subject 102 may thus allow the patient interface 111 to be set to a height that allows the subject to adopt a leaning position which is more closely tailored to their needs, and which may be more comfortable and thus further reduce the prevalence of motion artifacts within the image of the eye 101 acquired by the ophthalmic imaging device 110. By way of an example, for subjects between the ages of 50 and 60 years, the difference between the height indicated by the value Xᵢ of the first indicator and the height of the subject's eye (or the imaging axis 114) when the subject's chin is resting on the chinrest at the target height h_{T} may be 75% of the difference for subjects who are less 50 years old, for subjects between the ages of 60 and 70 years, the difference between the height indicated by the value Xᵢ of the first indicator and the height of the subject's eye (or the imaging axis 114) when the subject's chin is resting on the chinrest at the target height h_{T} may be 50% of the difference for subjects who are less 50 years old, and for subjects above the age of 70 years, the difference between the height indicated by the value Xᵢ of the first indicator and the height of the subject's eye (or the imaging axis 114) when the subject's chin is resting on the chinrest at the target height h_{T} may be 30% of the difference for subjects who are less 50 years old.

The mapping M may, as in the present example embodiment, also be dependent on the distance d_{DS} of the subject 102 from the contact surface 112, when the subject 102 is in the position beside the ophthalmic imaging device 110 such that, for each value of the first indicator, the respective target height h_{T} indicated by the corresponding value of the second indicator decreases with increasing distance d_{DS}. As the distance d_{DS} of the subject 102 from the contact surface 112 increases, the amount by which the subject 102 must lean forward to contact the contact surface 112 increases so the target height h_{T} at which contact surface 112 should decrease to account for the arcuate trajectory of the head 103 as the subject 102 leans forward (as shown in Figure 3C). This allows the target height h_{T} of the contact surface 112 to account for differing start positions of the subject 102, as dictated by the position of the seat 400. This is particularly useful where the seat 400 is a wheelchair, as it may be time-consuming and inaccurate to attempt to place the wheelchair in a predetermined position relative to the ophthalmic imaging device 110 each time the ophthalmic imaging device 110 is to be used. As noted above, the distance sensor 136-1 may, as in the present example embodiment, be located beside the patient interface 111 so as to measure the distance d_{DS} between the distance sensor 136-1, and thus equivalently between the contact surface 112 of the patient interface 111, and the head 103 or trunk of the subject 102.

Where the camera 132 is arranged to acquire the image 138 when the subject 102 is sitting in a seated position on a seat 400 beside the ophthalmic imaging device 110, as in the present example embodiment, the mapping M may be dependent on a height of the seat 400 such that, for each value of the first indicator, the respective target height h_{T} indicated by the corresponding value of the second indicator increases with increasing height of the seat 400. For a given value of the first indicator, the higher the seat 400, the shorter the spine of the subject 102 is likely to be. As the subject 102 pivots about their hips when leaning forward from a seated position, a shorter spine would provide a higher hip pivot point than a longer spine. Accordingly, the target height h_{T} indicated by the value of the second indicator increases with increasing height of the seat 400, to reflect the smaller drop in the height of the head of subject having a shorter spine when the subject leans forward, as compared to a subject having a longer spine. This may allow the processor 134 to determine a target height h_{T} of the contact surface 112 that does not require a subject having a relatively short spine to lean forward unnecessarily far, and may thus aid in setting a comfortable position for the subject 102 during the imaging of the eye 101 by the ophthalmic imaging device 110. This may further reduce the prevalence of motion artifacts within the image of the eye 101 acquired by the ophthalmic imaging device 110.

Accordingly, in process S20 the processor 134 may, as in the present example embodiment, use the received indication I_{age} of the age of the subject 102, the acquired indication I_{dist} of the distance d_{DS}, and the acquired indication I_{seat_h} of the height of the seat 400, each as described above with reference to Figure 2, and the at least one mapping M to map the value of the first indicator to the value of the second indicator. However, each of the above-described dependencies of the mapping M are optional improvements to the mapping M, and one or more of these may be omitted. For example, the mapping M may be dependent on the height of the seat 400 and not the distance d_{DS} of the subject 102 from the contact surface 112 and the age of the subject 102, so the processor 134 may only use the acquired indication I_{seat_h} of the height of the seat 400 and the mapping M to map the value of the first indicator to the value of the second indicator. In such a case, the processor 134 may not receive the indication I_{age} of the age of the subject 102 and may not acquire the indication I_{dist} of the distance d_{DS} as described with respect to Figure 2.

In process S30 of Figure 5, the processor 220 generates the first control signal S₁ for adjusting the height h at which the contact surface 112 is disposed to the target height h_{T} indicated by the value of the second indicator. The first control signal S₁ may be used to adjust the height h at which the contact surface 112 is disposed to the target height h_{T} as described above with respect to Figure 2.

Figure 7 is a flow diagram illustrating a process by which the processor 134 may, as in the present example embodiment, generate the second control signal S₂ for moving the contact surface 112 along the lateral axis (x-axis) to a target lateral position at distance d_{T} at which the contact surface 112 is to contact the head 103 of the subject 102 during the imaging of the eye 101 of the subject 102 by the ophthalmic imaging device 110. The process shown in Figure 7 may be performed in parallel with the process shown in Figure 5, although it may alternatively be performed after the process in Figure 5 has finished.

In process S100 of Figure 7, the processor 134 receives from the distance sensor 136-1 a value of a third indicator, which is indicative of a separation along the lateral axis (x-axis) between the contact surface 112 and a first lateral position at which the head 103 is disposed when the subject 102 is sitting in the seated position on the seat 400 beside the ophthalmic imaging device 110.

In an alternative to process S100 of Figure 7, which may be performed in example embodiments where the control system 130 comprises a stereo vision system having two cameras with parallel optical axes that are separated from one another, where the stereo vision system is arranged to acquire stereoscopic images of the head 103 of the subject 102, the processor 134 may process the images to generate a value of a third indicator which is indicative of a separation along the lateral axis (x-axis) between the contact surface 112 and a first lateral position at which the imaged portion 104 of the head 103 is disposed when the subject 102 is sitting in the seated position on the seat 400 beside the ophthalmic imaging device 110. The processor 134 may estimate the distance of the subject 102 from the cameras (hence from the contact surface 112) by processing the images using well-known techniques for distance estimation from stereo vision.

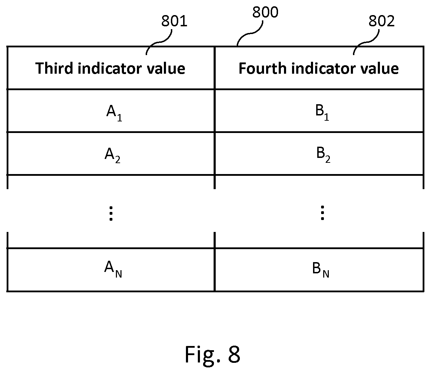

In process S200 of Figure 7, the processor 134 uses one or more second mappings to map the value of the third indicator to a value of a fourth indicator, which is indicative of the target lateral position at distance d_{T} at which the contact surface 112 is to contact the head 103 of the subject 102 during the imaging of the eye 101 of the subject 102 by the ophthalmic imaging device 110. The one or more second mappings map values of the third indication to corresponding values of the fourth indicator. Figure 8 is a schematic illustration of an example second mapping in the form of a table 800. The table 800 comprises a first column 801 with values A₁, A₂, ... A_{N} of the third indicator and a second column 802 with corresponding values B₁, B₂, ... B_{N} of the fourth indicator. However, the form of the second mapping is not so limited, and it may alternatively be provided as a function of multiple variables or a multi-dimensional look-up table, for example.

It should be noted that the first mapping(s) and the second mapping(s) may form part of a common mapping. That is, in some example embodiments, one or more mappings may receive a value of the first indicator and a value of the third indicator received as inputs, and output a value of the second indicator and a value of the fourth indicator. In this case, the mapping of the value of the first indicator to a value of the second indicator may be a function of the value of the third indicator. Additionally or alternatively, the mapping of the value of the third indicator to a value of the fourth indicator may be a function of the value of the first indicator

The at least one second mapping may similarly be dependent on the age of the subject 102 such that, for each value of the third indicator, the respective target lateral position at distance d_{T} indicated by the corresponding value of the fourth indicator comes closer to the first lateral position with increasing age. The processor 134 may thus use the received indication I_{age} of the age of the subject 102 as described with reference to Figure 2 and the at least one second mapping to map the value of the third indicator to the value of the fourth indicator.

The at least one second mapping may similarly be dependent on the height of the seat 400 such that, for each value of the third indicator, the respective target lateral position at distance d_{T} indicated by the corresponding value of the fourth indicator comes closer to the first lateral position with increasing height of the seat 400. The processor 134 may thus use the acquired indication I_{seat_h} of the height of the seat 400 as described with reference to

Figure 2 and the at least one second mapping to map the value of the third indicator to the value of the fourth indicator.

In process S300 of Figure 7, the processor 134 generates a second control signal S₂ for moving the contact surface 112 to the target lateral position indicated by the value of a fourth indicator. The second control signal S₂ may be used to move the contact surface 112 to the target lateral position indicated by the value of a fourth indicator as described above with reference to Figure 2.

In the foregoing description, example aspects are described with reference to several example embodiments. Accordingly, the specification should be regarded as illustrative, rather than restrictive. Similarly, the figures illustrated in the drawings, which highlight the functionality and advantages of the example embodiments, are presented for example purposes only. The architecture of the example embodiments is sufficiently flexible and configurable, such that it may be utilized in ways other than those shown in the accompanying figures.

Some aspects of the examples presented herein, such as the functions of the processor 134, may be provided as a computer program, or software, such as one or more programs having instructions or sequences of instructions, included or stored in an article of manufacture such as a machine-accessible or machine-readable medium, an instruction store, or computer-readable storage device, each of which can be non-transitory, in one example embodiment. The program or instructions on the non-transitory machine-accessible medium, machine-readable medium, instruction store, or computer-readable storage device, may be used to program a computer system or other electronic device. The machine- or computer-readable medium, instruction store, and storage device may include, but are not limited to, floppy diskettes, optical disks, and magneto-optical disks or other types of media/machine-readable medium/instruction store/storage device suitable for storing or transmitting electronic instructions. The techniques described herein are not limited to any particular software configuration. They may find applicability in any computing or processing environment. The terms "computer-readable", "machine-accessible medium", "machine-readable medium", "instruction store", and "computer-readable storage device" used herein shall include any medium that is capable of storing, encoding, or transmitting instructions or a sequence of instructions for execution by the machine, computer, or computer processor and that causes the machine/computer/computer processor to perform any one of the methods described herein. Furthermore, it is common in the art to speak of software, in one form or another (e.g., program, procedure, process, application, module, unit, logic, and so on), as taking an action or causing a result. Such expressions are merely a shorthand way of stating that the execution of the software by a processing system causes the processor to perform an action to produce a result.

Some or all of the functionality of the processor 134 may also be implemented by the preparation of application-specific integrated circuits, field-programmable gate arrays, or by interconnecting an appropriate network of conventional component circuits.

A computer program product may be provided in the form of a storage medium or media, instruction store(s), or storage device(s), having instructions stored thereon or therein which can be used to control, or cause, a computer or computer processor to perform any of the procedures of the example embodiments described herein. The storage medium/instruction store/storage device may include, by example and without limitation, an optical disc, a ROM, a RAM, an EPROM, an EEPROM, a DRAM, a VRAM, a flash memory, a flash card, a magnetic card, an optical card, nanosystems, a molecular memory integrated circuit, a RAID, remote data storage/archive/warehousing, and/or any other type of device suitable for storing instructions and/or data.

Stored on any one of the computer-readable medium or media, instruction store(s), or storage device(s), some implementations include software for controlling both the hardware of the system and for enabling the system or microprocessor to interact with a human user or other mechanism utilizing the results of the example embodiments described herein. Such software may include without limitation device drivers, operating systems, and user applications. Ultimately, such computer-readable media or storage device(s) further include software for performing example aspects of the invention, as described above.

Included in the programming and/or software of the system are software modules for implementing the procedures described herein. In some example embodiments herein, a module includes software, although in other example embodiments herein, a module includes hardware, or a combination of hardware and software.

While various example embodiments of the present invention have been described above, it should be understood that they have been presented by way of example, and not limitation. It will be apparent to persons skilled in the relevant art(s) that various changes in form and detail can be made therein. Thus, the present invention should not be limited by any of the above-described example embodiments, but should be defined only in accordance with the following claims. It is to be understood that any procedures recited in the claims need not be performed in the order presented.

While this specification contains many specific embodiment details, these should not be construed as limitations on the scope of any inventions or of what may be claimed, but rather as descriptions of features specific to particular embodiments described herein. Certain features that are described in this specification in the context of separate embodiments can also be implemented in combination in a single embodiment. Conversely, various features that are described in the context of a single embodiment can also be implemented in multiple embodiments separately or in any suitable subcombination.

In certain circumstances, multitasking and parallel processing may be advantageous. Moreover, the separation of various components in the embodiments described above should not be understood as requiring such separation in all embodiments, and the described program components and systems can generally be integrated together in a single software product or packaged into multiple software products.

Although many of the examples presented herein involve specific combinations of apparatus or software elements, those elements may be combined in other ways to accomplish the same objectives. Acts, elements and features discussed only in connection with one embodiment are not intended to be excluded from a similar role in other embodiments or embodiments.

## Claims

1. A control system (130) arranged to generate a control signal (S₁) for adjusting a height (h) at which a height-adjustable contact surface (112) of an ophthalmic imaging device (110) is disposed to a target height (h_{T}) at which the contact surface (112) is to contact a head (103) of a subject (102) during imaging of an eye (101) of the subject (102) by the ophthalmic imaging device (110), the control system (130) comprising:
at least one camera (132) arranged to acquire one or more images (138) of at least a portion (104) of the head (103) of the subject (102) where the subject is in a position beside the ophthalmic imaging device (110) for the head (103) to be brought into contact with the contact surface (112) when the contact surface is at the target height (h_{T}); and
a processor (134) arranged to:
process the one or more images (138) to generate a value of a first indicator which is indicative of a height at which the at least a portion of the head (103) was disposed when the one or more images (138) were acquired;
use at least one mapping (M) to map the value of the first indicator to a value of a second indicator which is indicative of the target height (h_{T}) at which the contact surface (112) is to contact the head (103) during the imaging; and
generate a control signal for adjusting the height (h) at which the contact surface (112) is disposed to the target height (h_{T}) indicated by the value of the second indicator,
wherein the value of the first indicator is indicative of a height at which the eye (101) of the subject (102) was disposed when the one or more images (138) were acquired, and
wherein the at least one mapping (M) maps each value of the first indicator to a corresponding value of the second indicator such that a height of an imaging axis (114) of the ophthalmic imaging device (110) when the contact surface (112) is disposed at the target height (h_{T}) indicated by the value of the second indicator is smaller than the height indicated by the value of first indicator.

2. The control system (130) of Claim 1, wherein
the height (h) of the contact surface (112) is adjustable by a user of the control system (130),
the control system (130) further comprises a user interface (113) for providing the user with instructions for adjusting the height (h) of the contact surface (112), and
the processor (134) is arranged to control the user interface (113), using the generated control signal (S₁), to provide the user with instructions for adjusting the height (h) of the contact surface (112) to the target height (h_{T}).

3. The control system (130) of Claim 1, wherein the height (h) of the contact surface (112) of the ophthalmic imaging device (110) is automatically adjustable by a height adjustment mechanism, and the generated control signal (S₁) is arranged to cause the height adjustment mechanism to automatically adjust the height (h) of the contact surface (112) to the target height (h_{T}).

4. The control system (130) of any preceding claim, wherein
the at least one mapping (M) is dependent on an age of the subject (102) such that, for each value of the first indicator, the respective target height (h_{T}) indicated by the corresponding value of the second indicator increases with increasing age of the subject (102), and
the processor (134) is further arranged to receive an indication (I_{age}) of the age of the subject (102), and to use the received indication (I_{age}) of the age and the at least one mapping to map the value of the first indicator to the value of the second indicator.

5. The control system (130) of any preceding claim, wherein
the at least one mapping (M) is dependent on a distance (d_{DS}) of the subject (102) from the contact surface (112) where the subject (102) is in the position beside the ophthalmic imaging device (110) such that, for each value of the first indicator, the respective target height (h_{T}) indicated by the corresponding value of the second indicator decreases with increasing distance (d_{DS}), and
the processor (134) is further arranged to acquire an indication (I_{dist}) of the distance (d_{DS}), and to use the acquired indication (I_{dist}) of the distance (d_{DS}) and the at least one mapping (M) to map the value of the first indicator to the value of the second indicator.

6. The control system (130) of Claim 5, wherein the processor (134) is arranged to acquire the indication (I_{dist}) of the distance (d_{DS}) by processing at least some of the one or more images (138) acquired by the at least one camera (132).

7. The control system (130) of Claim 5, further comprising:
a distance sensor (136-1) arranged to measure the distance (d_{DS}) of the subject (102) from the contact surface (112) where the subject (102) is in the position beside the ophthalmic imaging device (110),
wherein the processor (134) is arranged to acquire the indication (I_{dist}) of the distance (d_{DS}) by receiving the measured distance from the distance sensor (136-1).

8. The control system (130) of any preceding claim, wherein
the at least one camera (132) is arranged to acquire the one or more images (138) when the subject (102) is sitting in a seated position on a seat (400) beside the ophthalmic imaging device (110),
the at least one mapping (M) is dependent on a height of the seat (400) such that, for each value of the first indicator, the respective target height (h_{T}) indicated by the corresponding value of the second indicator increases with increasing height of the seat, and
the processor (134) is further arranged to acquire an indication (I_{seat_h}) of the height of the seat (400), and to use the acquired indication (I_{seat_h}) of the height of the seat (400) and the at least one mapping (M) to map the value of the first indicator to the value of the second indicator.

9. The control system (130) of any preceding claim, wherein
the contact surface (112) is movable in a lateral direction (x) towards the head (103) of the subject (102),
the control system (130) is further arranged to generate a second control signal (S₂) for moving the contact surface (112) along the lateral axis (x) to a target lateral position at which the contact surface (112) is to contact the head (103) of the subject (102) during the imaging of the eye (101) of the subject (102) by the ophthalmic imaging device (110), and
the processor (134) is further arranged to:
process the one or more images (138) to generate a value of a third indicator which is indicative of a separation along the lateral axis (x) between the contact surface (112) and a first lateral position at which the at least a portion of the head (103) is disposed when the subject (102) is sitting in the seated position on the seat (400) beside the ophthalmic imaging device (110);
use at least one second mapping (M) to map the value of the third indicator to a value of a fourth indicator which is indicative of the target lateral position at which the contact surface (112) is to contact the head (103) of the subject (102) during the imaging of the eye (101) of the subject (102) by the ophthalmic imaging device (110); and
generate a second control signal (S₂) for moving the contact surface (112) to the target lateral position indicated by the value of a fourth indicator.

10. The control system (130) of any of Claims 1 to 8, wherein
the contact surface (112) is movable in a lateral direction (x) towards the head (103) of the subject (102),
the control system (130) is further arranged to generate a second control signal (S₂) for moving the contact surface (112) along the lateral axis (x) to a target lateral position at which the contact surface (112) is to contact the head (103) of the subject (102) during the imaging of the eye (101) of the subject (102) by the ophthalmic imaging device (110),
the control system (130) further comprises a distance sensor (136-1) arranged to generate a value of a third indicator which is indicative of a separation along the lateral axis (x) between the contact surface (112) and a first lateral position at which the at least a portion (104) of the head (103) is disposed when the subject (102) is sitting in the seated position on the seat (400) beside the ophthalmic imaging device (110), and
the processor (134) is further arranged to:
use at least one second mapping (M) to map the value of the third indicator to a value of a fourth indicator which is indicative of a target lateral position at which the contact surface (112) is to contact the head (103) of the subject (102) during the imaging of the eye (101) of the subject (102) by the ophthalmic imaging device (110); and
generate a second control signal (S₂) for moving the contact surface (112) along the lateral axis (x) to the target lateral position indicated by the value of a fourth indicator.

11. The control system (130) of Claim 9 or Claim 10, wherein
the at least one second mapping (M) maps values of the third indicator to corresponding values of the fourth indicator,
the at least one second mapping (M) is dependent on an age of the subject (102) such that, for each value of the first indicator, the respective target lateral position indicated by the corresponding value of the fourth indicator comes closer to the first lateral position with increasing age, and
the processor (134) is further arranged to receive an indication (I_{age}) of the age of the subject (102), and to use the received indication (I_{age}) of the age of the subject (102) and the at least one second mapping (M) to map the value of the third indicator to the value of the fourth indicator.

12. The control system (130) of any preceding claim, wherein a difference between the height indicated by the value of the first indicator and the height of the imaging axis (114) when the head (103) of the subject (102) contacts the height-adjustable contact surface (112) of the ophthalmic imaging device (110) is set to a predetermined value which is within a range of 2 cm to 10 cm.

13. A system (100) for imaging an eye (101) of a subject (102), comprising:
an ophthalmic imaging device (110) arranged to image the eye (101) of the subject (102), the ophthalmic imaging device (110) comprising a height-adjustable contact surface (112) arranged to contact a head (103) of the subject (102) during the imaging of the eye (101);
a movement mechanism (120) arranged to adjust a height (h) relative to ground level, at which the contact surface (112) is disposed, to a target height (h_{T}) at which the contact surface (112) is to contact the head (103) of the subject (102) during the imaging of the eye (101); and
the control system (130) of any preceding claim, which is arranged to generate a control signal (S1) for controlling the movement mechanism (120) to adjust the height (h) to the target height (h_{T}).

14. The system (100) of Claim 13, wherein the movement mechanism (120) comprises a height-adjustable table (300) which is arranged to support the ophthalmic imaging device (110).

15. The system (100) of Claim 14, further comprising at least one sensor (136-1, 136-2, 136-3) of an object, each being one of a distance sensor arranged to measure a distance to the object (102; 400) and a proximity sensor arranged to detect the object (102; 400) when the object is within a detection range of the proximity sensor, wherein the at least one sensor (136-1, 136-2, 136-3) and the control system (130) are arranged to determine whether at least one of the height-adjustable table (300) or the ophthalmic imaging device (110) has been moved to within a predetermined distance of the object and, in response to determining that the at least one of the height-adjustable table (300) or the ophthalmic imaging device (110) has been moved to within the predetermined distance of the object, generating an instruction to stop an adjustment of the height (h) to the target height (h_{T}) being made by the movement mechanism (120).

## Patentansprüche

1. Ein Steuersystem (130) eingerichtet zum Generieren eines Steuersignals (S₁) zum Justieren einer Höhe (h), auf der eine höhenjustierbare Kontaktfläche (112) einer ophthalmischen Bildgebungsvorrichtung (110) auf eine Zielhöhe (h_{T}) angeordnet ist, auf der die Kontaktfläche (112) einen Kopf (103) eines Subjekts (102), während einer Bildgebung eines Auges (101) des Subjekts (102) durch die ophthalmische Bildgebungsvorrichtung (110), kontaktieren soll, wobei das Steuersystem (130) umfasst:
mindestens eine Kamera (132), eingerichtet zum Erfassen eines oder mehrerer Bilder (138) von mindestens einem Abschnitt (104) des Kopfes (103) des Subjekts (102), wobei sich das Subjekt in einer Position neben der ophthalmischen Bildgebungsvorrichtung (110) befindet, um den Kopf (103) mit der Kontaktfläche (112) in Kontakt zu bringen, wenn sich die Kontaktfläche auf der Zielhöhe (h_{T}) befindet; und
einen Prozessor (134) eingerichtet zum:
Verarbeiten des einen oder der mehreren Bilder (138), um einen ersten Indikatorwert zu generieren, der indikativ ist für eine Höhe, auf der mindestens ein Abschnitt des Kopfes (103) angeordnet war, als das eine oder die mehreren Bilder (138) erfasst wurden;
Verwenden von mindestens einer Abbildung (M), um den ersten Indikatorwert auf einen zweiten Indikatorwert abzubilden, der indikativ ist für die Zielhöhe (h_{T}), auf der die Kontaktfläche (112) den Kopf (103) während der Bildgebung kontaktieren soll; und
Generieren eines Steuersignals zum Justieren der Höhe (h), auf der die Kontaktfläche (112) angeordnet ist, auf die Zielhöhe (h_{T}), die durch den zweiten Indikatorwert indiziert ist,
wobei der Indikatorwert indikativ ist für eine Höhe, auf der das Auge (101) des Subjekts (102) angeordnet war, als das eine oder die mehreren Bilder (138) erfasst wurden, und
wobei die mindestens eine Abbildung (M) jeden ersten Indikatorwert auf einen entsprechenden zweiten Indikatorwert derart abbildet, dass eine Höhe einer Bildgebungsachse (114) der ophthalmischen Bildgebungsvorrichtung (110), wenn die Kontaktfläche (112) auf der Zielhöhe (h_{T}) angeordnet ist, die durch den zweiten Indikatorwert indiziert ist, kleiner ist als die Höhe, die durch den ersten Indikatorwert indiziert ist.

2. Das Steuersystem (130) nach Anspruch 1, wobei
die Höhe (h) der Kontaktfläche (112) durch einen Benutzer des Steuersystems (130) justierbar ist,
das Steuersystem (130) ferner eine Benutzerschnittstelle (113) umfasst zum Bereitstellen von Instruktionen an den Benutzer zum Justieren der Höhe (h) der Kontaktfläche (112), und
der Prozessor (134) eingerichtet ist zum Steuern der Benutzerschnittstelle (113) unter Verwendung des generierten Steuersignals (S₁), um dem Benutzer Instruktionen bereitzustellen zum Justieren der Höhe (h) der Kontaktfläche (112) auf die Zielhöhe (h_{T}).

3. Das Steuersystem (130) nach Anspruch 1, wobei die Höhe (h) der Kontaktfläche (112) der ophthalmischen Bildgebungsvorrichtung (110) automatisch durch einen Höhenjustiermechanismus justierbar ist, und das generierte Steuersignal (S₁) eingerichtet ist, um den Höhenjustiermechanismus zu veranlassen, die Höhe (h) der Kontaktfläche (112) automatisch auf die Zielhöhe (h_{T}) zu justieren.

4. Das Steuersystem (130) nach einem der vorhergehenden Ansprüche, wobei
die mindestens eine Abbildung (M) von einem Alter des Subjekts (102) abhängig ist, sodass für jeden ersten Indikatorwert die jeweilige Zielhöhe (h_{T}), die durch den entsprechenden zweiten Indikatorwert indiziert ist, mit zunehmendem Alter des Subjekts (102) ansteigt, und
der Prozessor (134) ferner eingerichtet ist zum Empfangen einer Indikation (I_{age}) des Alters des Subjekts (102) und zum Verwenden der empfangenen Indikation (I_{age}) des Alters und der mindestens einen Abbildung, um den ersten Indikatorwert auf den zweiten Indikatorwert abzubilden.

5. Das Steuersystem (130) nach einem der vorhergehenden Ansprüche, wobei
die mindestens eine Abbildung (M) von einer Distanz (d_{DS}) des Subjekts (102) von der Kontaktfläche (112) abhängig ist, in der sich das Subjekt (102) in der Position neben der ophthalmischen Bildgebungsvorrichtung (110) befindet, sodass für jeden ersten Indikatorwert die jeweilige Zielhöhe (h_{T}), die durch den entsprechenden zweiten Indikatorwert indiziert ist, mit zunehmender Distanz (d_{DS}) abnimmt, und
der Prozessor (134) ferner eingerichtet ist zum Empfangen einer Indikation (I_{age}) der Distanz (d_{DS}) und zum Verwenden der empfangenen Indikation (I_{age}) der Distanz (d_{DS}) und der mindestens einen Abbildung, um den ersten Indikatorwert auf den zweiten Indikatorwert abzubilden.

6. Das Steuersystem (130) nach Anspruch 5, wobei der Prozessor (134) eingerichtet ist zum Erfassen der Indikation (I_{dist}) der Distanz (d_{DS}) durch Verarbeiten von mindestens einigen des einen oder der mehreren Bilder (138), die durch die mindestens eine Kamera (132) erfasst wurden.

7. Das Steuersystem (130) nach Anspruch 5, ferner umfassend:
einen Distanzsensor (136-1), eingerichtet zum Messen der Distanz (d_{DS}) des Subjekts (102) von der Kontaktfläche (112), wobei sich das Subjekt (102) in der Position neben der ophthalmischen Bildgebungsvorrichtung (110) befindet,
wobei der Prozessor (134) eingerichtet ist zum Erfassen der Indikation (I_{dist}) der Distanz (d_{DS}) durch Empfangen der gemessenen Distanz von dem Distanzsensor (136-1).

8. Das Steuersystem (130) nach einem der vorhergehenden Ansprüche, wobei
die mindestens eine Kamera (132) eingerichtet ist zum Erfassen des einen oder der mehreren Bilder (138), wenn das Subjekt (102) in einer sitzenden Position auf einem Sitz (400) neben der ophthalmischen Bildgebungsvorrichtung (110) sitzt, die mindestens eine Abbildung (M) von einer Höhe des Sitzes (400) abhängig ist, sodass für jeden ersten Indikatorwert die jeweilige Zielhöhe (h_{T}), die durch den entsprechenden zweiten Indikatorwert indiziert ist, mit zunehmender Höhe des Sitzes ansteigt, und
der Prozessor (134) ferner eingerichtet ist zum Erfassen einer Indikation (I_{seat_h}) der Höhe des Sitzes (400) und zum Verwenden der erfassten Indikation (I_{seat_h}) der Höhe des Sitzes (400) und der mindestens einen Abbildung (M), um den ersten Indikatorwert auf den zweiten Indikatorwert abzubilden.

9. Das Steuersystem (130) nach einem der vorhergehenden Ansprüche, wobei
die Kontaktfläche (112) in einer lateralen Richtung (x) in Richtung zum Kopf (103) des Subjekts (102) bewegbar ist,
das Steuersystem (130) ferner eingerichtet ist zum Generieren eines zweiten Steuersignals (S₂) zum Bewegen der Kontaktfläche (112) entlang der lateralen Achse (x) auf eine laterale Zielposition, auf der die Kontaktfläche (112) den Kopf (103) des Subjekts (102), während der Bildgebung des Auges (101) des Subjekts (102) durch die ophthalmische Bildgebungsvorrichtung (110), kontaktieren soll, und
der Prozessor (134) ferner eingerichtet ist zum:
Verarbeiten des einen oder der mehreren Bilder (138), um einen dritten Indikatorwert zu generieren, der indikativ ist für einen Abstand entlang der lateralen Achse (x) zwischen der Kontaktfläche (112) und einer ersten lateralen Position, auf der mindestens ein Abschnitt des Kopfes (103) angeordnet ist, wenn das Subjekt (102) in der sitzenden Position auf dem Sitz (400) neben der ophthalmischen Bildgebungsvorrichtung (110) sitzt;
Verwenden mindestens einer zweiten Abbildung (M), um den dritten Indikatorwert auf einen vierten Indikatorwert abzubilden, der indikativ ist für die laterale Zielposition, auf der die Kontaktfläche (112) den Kopf (103) des Subjekts (102), während der Bildgebung des Auges (101) des Subjekts (102) durch die ophthalmische Bildgebungsvorrichtung (110), kontaktieren soll; und
Generieren eines zweiten Steuersignals (S₂) zum Bewegen der Kontaktfläche (112) auf die laterale Zielposition, die durch den vierten Indikatorwert indiziert ist.

10. Das Steuersystem (130) nach einem der Ansprüche 1 bis 8, wobei
die Kontaktfläche (112) in einer lateralen Richtung (x) in Richtung zum Kopf (103) des Subjekts (102) bewegbar ist,
das Steuersystem (130) ferner eingerichtet ist zum Generieren eines zweiten Steuersignals (S₂) zum Bewegen der Kontaktfläche (112) entlang der lateralen Achse (x) auf eine laterale Zielposition, auf der die Kontaktfläche (112) den Kopf (103) des Subjekts (102), während der Bildgebung des Auges (101) des Subjekts (102) durch die ophthalmische Bildgebungsvorrichtung (110), kontaktieren soll,
das Steuersystem (130) ferner einen Distanzsensor (136-1) umfasst, eingerichtet zum Generieren eines dritten Indikatorwerts, der indikativ ist für einen Abstand entlang der lateralen Achse (x) zwischen der Kontaktfläche (112) und einer ersten lateralen Position, auf der mindestens ein Abschnitt (104) des Kopfes (103) angeordnet ist, wenn das Subjekt (102) in der sitzenden Position auf dem Sitz (400) neben der ophthalmischen Bildgebungsvorrichtung (110) sitzt, und
der Prozessor (134) ferner eingerichtet ist zum:
Verwenden mindestens einer zweiten Abbildung (M), um den dritten Indikatorwert auf einen vierten Indikatorwert abzubilden, der indikativ ist für die laterale Zielposition, auf der die Kontaktfläche (112) den Kopf (103) des Subjekts (102) während der Bildgebung des Auges (101) des Subjekts (102) durch die ophthalmische Bildgebungsvorrichtung (110) kontaktieren soll; und
Generieren eines zweiten Steuersignals (S2) zum Bewegen der Kontaktfläche (112) entlang der lateralen Achse (x) auf die laterale Zielposition, die durch den Wert eines vierten Indikators indiziert ist.
die mindestens eine Abbildung (M) von einer Höhe des Sitzes (400) abhängig ist, sodass für jeden ersten Indikatorwert die jeweilige Zielhöhe (h_{T}), die durch den entsprechenden zweiten Indikatorwert indiziert ist, mit zunehmender Höhe des Sitzes ansteigt, und

11. Das Steuersystem (130) nach Anspruch 9 oder Anspruch 10, wobei
die mindestens eine zweite Abbildung (M) welche die dritten Indikatorwerte auf entsprechende vierten Indikatorwerte abbildet,
die mindestens eine zweite Abbildung (M) von einem Alter des Subjekts (102) abhängig ist, sodass für jeden ersten Indikatorwert die jeweilige laterale Zielposition, die durch den entsprechenden vierten Indikatorwert indiziert ist, der ersten lateralen Position mit zunehmendem Alter näher kommt, und
der Prozessor (134) ferner eingerichtet ist zum Empfangen einer Indikation (I_{age}) des Alters des Subjekts (102) und zum Verwenden der empfangenen Indikation (I_{age}) des Alters des Subjekts (102) und der mindestens einen zweiten Abbildung (M), um den dritten Indikatorwert auf den vierten Indikatorwert abzubilden.

12. Das Steuersystem (130) nach einem der vorhergehenden Ansprüche, wobei eine Differenz zwischen der Höhe, die durch den ersten Indikatorwert indiziert ist, und der Höhe der Bildgebungsachse (114), wenn der Kopf (103) des Subjekts (102) die höhenjustierbare Kontaktfläche (112) der ophthalmischen Bildgebungsvorrichtung (110) kontaktiert, auf einen vorbestimmten Wert eingestellt ist, der innerhalb eines Bereichs von 2 cm bis 10 cm liegt.

13. Ein System (100) zur Bildgebung eines Auges (101) eines Subjekts (102), umfassend:
eine ophthalmische Bildgebungsvorrichtung (110), eingerichtet zur Bildgebung des Auges (101) des Subjekts (102), wobei die ophthalmische Bildgebungsvorrichtung (110) eine höhenjustierbare Kontaktfläche (112) umfasst, die eingerichtet ist zum Kontaktieren eines Kopfes (103) des Subjekts (102) während der Bildgebung des Auges (101);
einen Bewegungsmechanismus (120), eingerichtet zum Justieren einer Höhe (h) relativ zu einem Bodenniveau, auf der die Kontaktfläche (112) angeordnet ist, auf eine Zielhöhe (hT), auf der die Kontaktfläche (112) den Kopf (103) des Subjekts (102) während der Bildgebung des Auges (101) kontaktieren soll; und
das Steuersystem (130) nach einem der vorhergehenden Ansprüche, eingerichtet zum Generieren eines Steuersignals (S1) zum Steuern des Bewegungsmechanismus (120), um die Höhe (h) auf die Zielhöhe (h_{T}) zu justieren.

14. Das System (100) nach Anspruch 13, wobei der Bewegungsmechanismus (120) einen höhenjustierbaren Tisch (300) umfasst, der eingerichtet ist zum Tragen der ophthalmischen Bildgebungsvorrichtung (110).

15. Das System (100) nach Anspruch 14, ferner umfassend mindestens einen Sensor (136-1, 136-2, 136-3) eines Objekts, wobei jeder entweder ein Distanzsensor, eingerichtet zum Messen einer Distanz zu dem Objekt (102; 400), oder ein Näherungssensor, eingerichtet zum Detektieren des Objekts (102; 400), wenn das Objekt innerhalb einer Detektionsnähe des Näherungssensors ist, ist, wobei der mindestens eine Sensor (136-1, 136-2, 136-3) und das Steuersystem (130) eingerichtet sind zum Bestimmen, ob mindestens einer aus dem höhenjustierbaren Tisch (300) oder der ophthalmischen Bildgebungsvorrichtung (110) auf innerhalb einer vorbestimmten Distanz zum Objekt bewegt wurde, und in Reaktion auf das Bestimmen, dass der mindestens eine aus dem höhenjustierbaren Tisch (300) oder der ophthalmischen Bildgebungsvorrichtung (110) auf innerhalb der vorbestimmten Distanz zum Objekt bewegt wurde, Generieren einer Instruktion zum Stoppen einer Justierung der Höhe (h) auf die Zielhöhe (h_{T}), die durch den Bewegungsmechanismus (120) vorgenommen wird.

## Revendications

1. Système de commande (130) agencé pour générer un signal de commande (S₁) pour ajuster une hauteur (h) à laquelle une surface de contact réglable en hauteur (112) d'un dispositif d'imagerie ophtalmique (110) est positionnée à une hauteur cible (h_{T}) à laquelle la surface de contact (112) doit entrer en contact avec une tête (103) d'un sujet (102) pendant l'imagerie d'un œil (101) du sujet (102) par le dispositif d'imagerie ophtalmique (110), le système de commande (130) comprenant :
au moins une caméra (132) agencée pour acquérir une ou plusieurs images (138) d'au moins une partie (104) de la tête (103) du sujet (102) lorsque le sujet est dans une position à côté du dispositif d'imagerie ophtalmique (110) de manière à ce que la tête (103) soit amenée en contact avec la surface de contact (112) lorsque la surface de contact se trouve à la hauteur cible (h_{T}) ; et
un processeur (134) agencé pour :
traiter la ou les images (138) afin de générer une valeur d'un premier indicateur qui est indicative d'une hauteur à laquelle la au moins une partie de la tête (103) était disposée lorsque la ou les images (138) ont été acquises ;
utiliser au moins une correspondance (M) pour faire correspondre la valeur du premier indicateur à une valeur d'un deuxième indicateur qui est indicative de la hauteur cible (h_{T}) à laquelle la surface de contact (112) doit entrer en contact avec la tête (103) pendant l'imagerie ; et
générer un signal de commande pour ajuster la hauteur (h) à laquelle la surface de contact (112) est disposée à la hauteur cible (h_{T}) indiquée par la valeur du deuxième indicateur,
dans lequel la valeur du premier indicateur est indicative d'une hauteur à laquelle l'œil (101) du sujet (102) était disposé lorsque la ou les images (138) ont été acquises, et
dans lequel la au moins une correspondance (M) fait correspondre chaque valeur du premier indicateur à une valeur correspondante du deuxième indicateur de telle sorte que la hauteur d'un axe d'imagerie (114) du dispositif d'imagerie ophtalmique (110), lorsque la surface de contact (112) est disposée à la hauteur cible (h_{T}) indiquée par la valeur du deuxième indicateur, soit inférieure à la hauteur indiquée par la valeur du premier indicateur.

2. Système de commande (130) selon la revendication 1, dans lequel
la hauteur (h) de la surface de contact (112) est réglable par un utilisateur du système de commande (130),
le système de commande (130) comprend en outre une interface utilisateur (113) destinée à fournir à l'utilisateur des instructions pour ajuster la hauteur (h) de la surface de contact (112), et
le processeur (134) est agencé pour commander l'interface utilisateur (113), à l'aide du signal de commande généré (S1), afin de fournir à l'utilisateur des instructions pour ajuster la hauteur (h) de la surface de contact (112) à la hauteur cible (h_{T}).

3. Système de commande (130) selon la revendication 1, dans lequel la hauteur (h) de la surface de contact (112) du dispositif d'imagerie ophtalmique (110) est automatiquement réglable par un mécanisme de réglage de hauteur, et le signal de commande généré (S1) est agencé pour amener le mécanisme de réglage de hauteur à régler automatiquement la hauteur (h) de la surface de contact (112) à la hauteur cible (h_{T}).

4. Système de commande (130) selon l'une quelconque des revendications précédentes, dans lequel
la au moins une correspondance (M) dépend d'un âge du sujet (102) de telle sorte que, pour chaque valeur du premier indicateur, la hauteur cible respective (h_{T}) indiquée par la valeur correspondante du deuxième indicateur augmente avec l'âge du sujet (102), et
le processeur (134) est en outre agencé pour recevoir une indication (I_{age}) de l'âge du sujet (102), et pour utiliser l'indication reçue (I_{age}) de l'âge et la au moins une correspondance afin de faire correspondre la valeur du premier indicateur à la valeur du deuxième indicateur.

5. Système de commande (130) selon l'une quelconque des revendications précédentes, dans lequel
la au moins une correspondance (M) dépend d'une distance (d_{DS}) entre le sujet (102) et la surface de contact (112) lorsque le sujet (102) est dans la position à côté du dispositif d'imagerie ophtalmique (110), de telle sorte que, pour chaque valeur du premier indicateur, la hauteur cible respective (h_{T}) indiquée par la valeur correspondante du deuxième indicateur diminue à mesure que la distance (d_{DS}) augmente, et
le processeur (134) est en outre agencé pour acquérir une indication (I_{dist}) de la distance (d_{DS}), et pour utiliser l'indication acquise (I_{dist}) de la distance (d_{DS}) et la au moins une correspondance (M) afin de faire correspondre la valeur du premier indicateur à la valeur du deuxième indicateur.

6. Système de commande (130) selon la revendication 5, dans lequel le processeur (134) est agencé pour acquérir l'indication (I_{dist}) de la distance (d_{DS}) en traitant au moins certaines des une ou plusieurs images (138) acquises par la au moins une caméra (132).

7. Système de commande (130) selon la revendication 5, comprenant en outre :
un capteur de distance (136-1) agencé pour mesurer la distance (d_{DS}) du sujet (102) par rapport à la surface de contact (112) lorsque le sujet (102) est dans la position à côté du dispositif d'imagerie ophtalmique (110),
dans lequel le processeur (134) est agencé pour acquérir l'indication (I_{dist}) de la distance (d_{DS}) en recevant la distance mesurée provenant du capteur de distance (136-1).

8. Système de commande (130) selon l'une quelconque des revendications précédentes, dans lequel
la au moins une caméra (132) est agencée pour acquérir la ou les images (138) lorsque le sujet (102) est assis dans une position assise sur un siège (400) à côté du dispositif d'imagerie ophtalmique (110),
la au moins une correspondance (M) dépend de la hauteur du siège (400) de telle sorte que, pour chaque valeur du premier indicateur, la hauteur cible respective (h_{T}) indiquée par la valeur correspondante du deuxième indicateur augmente à mesure que la hauteur du siège augmente, et
le processeur (134) est en outre agencé pour acquérir une indication (I_{seat_h}) de la hauteur du siège (400), et pour utiliser l'indication acquise (I_{seat_h}) de la hauteur du siège (400) et la au moins une correspondance (M) afin de faire correspondre la valeur du premier indicateur à la valeur du deuxième indicateur.

9. Système de commande (130) selon l'une quelconque des revendications précédentes, dans lequel
la surface de contact (112) est mobile dans une direction latérale (x) vers la tête (103) du sujet (102),
le système de commande (130) est en outre agencé pour générer un deuxième signal de commande (S₂) destiné à déplacer la surface de contact (112) le long de l'axe latéral (x) vers une position latérale cible (d_{T}) à laquelle la surface de contact (112) doit entrer en contact avec la tête (103) du sujet (102) pendant l'imagerie de l'œil (101) du sujet (102) par le dispositif d'imagerie ophtalmique (110), et
le processeur (134) est en outre agencé pour :
traiter la ou les images (138) afin de générer une valeur d'un troisième indicateur qui est indicative d'une séparation le long de l'axe latéral (x) entre la surface de contact (112) et une première position latérale à laquelle au moins une partie de la tête (103) se trouve lorsque le sujet (102) est assis dans la position assise sur le siège (400) à côté du dispositif d'imagerie ophtalmique (110) ;
utiliser au moins une deuxième correspondance (M) pour mettre en correspondance la valeur du troisième indicateur avec une valeur d'un quatrième indicateur qui est indicative de la position latérale cible (d_{T}) à laquelle la surface de contact (112) doit entrer en contact avec la tête (103) du sujet (102) pendant l'imagerie de l'œil (101) du sujet (102) par le dispositif d'imagerie ophtalmique (110) ; et
générer un deuxième signal de commande (S₂) pour déplacer la surface de contact (112) vers la position latérale cible (d_{T}) indiquée par la valeur d'un quatrième indicateur.

10. Système de commande (130) selon l'une quelconque des revendications 1 à 8, dans lequel
la surface de contact (112) est mobile dans une direction latérale (x) vers la tête (103) du sujet (102),
le système de commande (130) est en outre agencé pour générer un deuxième signal de commande (S₂) pour déplacer la surface de contact (112) le long de l'axe latéral (x) vers une position latérale cible (d_{T}) à laquelle la surface de contact (112) doit entrer en contact avec la tête (103) du sujet (102) pendant l'imagerie de l'œil (101) du sujet (102) par le dispositif d'imagerie ophtalmique (110),
le système de commande (130) comprend en outre un capteur de distance (136-1) agencé pour générer une valeur d'un troisième indicateur qui est indicative d'une séparation le long de l'axe latéral (x) entre la surface de contact (112) et une première position latérale à laquelle la au moins une partie (104) de la tête (103) se trouve lorsque le sujet (102) est assis dans la position assise sur le siège (400) à côté du dispositif d'imagerie ophtalmique (110), et
le processeur (130) est en outre agencé pour :
utiliser au moins une deuxième correspondance (M) pour faire correspondre la valeur du troisième indicateur à une valeur d'un quatrième indicateur qui est indicative d'une position latérale cible (d_{T}) à laquelle la surface de contact (112) doit entrer en contact avec la tête (103) du sujet (102) pendant l'imagerie de l'œil (101) du sujet (102) par le dispositif d'imagerie ophtalmique (110) ; et
générer un deuxième signal de commande (S₂) pour déplacer la surface de contact (112) le long de l'axe latéral (x) jusqu'à la position latérale cible (d_{T}) indiquée par la valeur d'un quatrième indicateur.

11. Système de commande (130) selon la revendication 9 ou la revendication 10, dans lequel
la au moins une deuxième correspondance (M) met en correspondance des valeurs du troisième indicateur avec des valeurs correspondantes du quatrième indicateur,
la au moins une deuxième correspondance (M) dépend d'un âge du sujet (102) de telle sorte que, pour chaque valeur du premier indicateur, la position latérale cible respective (d_{T}) indiquée par la valeur correspondante du quatrième indicateur se rapproche de la première position latérale à mesure que l'âge augmente, et
le processeur (134) est en outre agencé pour recevoir une indication (I_{age}) de l'âge du sujet (102), et pour utiliser l'indication reçue (I_{age}) de l'âge du sujet (102) et la au moins une deuxième correspondance (M) pour faire correspondre la valeur du troisième indicateur à la valeur du quatrième indicateur.

12. Système de commande (130) selon l'une quelconque des revendications précédentes, dans lequel une différence entre la hauteur indiquée par la valeur du premier indicateur et la hauteur de l'axe d'imagerie (114) lorsque la tête (103) du sujet (102) entre en contact avec la surface de contact réglable en hauteur (112) du dispositif d'imagerie ophtalmique (110) est réglée à une valeur prédéterminée comprise dans une plage de 2 cm à 10 cm.

13. Système (100) destiné à l'imagerie d'un œil (101) d'un sujet (102), comprenant :
un dispositif d'imagerie ophtalmique (110) agencé pour réaliser une image de l'œil (101) du sujet (102), le dispositif d'imagerie ophtalmique (110) comprenant une surface de contact réglable en hauteur (112) agencée pour entrer en contact avec une tête (103) du sujet (102) pendant l'imagerie de l'œil (101) ;
un mécanisme de déplacement (120) agencé pour ajuster une hauteur (h) par rapport au niveau du sol à laquelle la surface de contact réglable en hauteur (112) est disposée à une hauteur cible (h_{T}) à laquelle la surface de contact (112) doit entrer en contact avec la tête (103) du sujet (102) pendant l'imagerie de l'œil (101) ; et
le système de commande (130) de l'une quelconque des revendications précédentes, qui est agencé pour générer un signal de commande (S1) pour commander le mécanisme de déplacement (120) pour ajuster la hauteur (h) à la hauteur cible (h_{T}).

14. Système (100) selon la revendication 13, dans lequel le mécanisme de déplacement (120) comprend une table réglable en hauteur (300) qui est agencée pour supporter le dispositif d'imagerie ophtalmique (110).

15. Système (100) selon la revendication 14, comprenant en outre au moins un capteur (136-1, 136-2, 136-3) d'un objet, chacun étant soit un capteur de distance agencé pour mesurer une distance par rapport à l'objet (102 ; 400), soit un capteur de proximité agencé pour détecter l'objet (102 ; 400) lorsque l'objet se trouve dans une plage de détection du capteur de proximité, dans lequel le ou les capteurs (136-1, 136-2, 136-3) et le système de commande (130) sont agencés pour déterminer si au moins un parmi la table réglable en hauteur (300) ou le dispositif d'imagerie ophtalmique (110) a été déplacé à une distance prédéterminée de l'objet et, en réponse à la détermination selon laquelle au moins un parmi la table réglable en hauteur (300) ou le dispositif d'imagerie ophtalmique (110) a été déplacé à une distance prédéterminée de l'objet, générer une instruction pour arrêter un réglage de la hauteur (h) à la hauteur cible (h_{T}) effectué par le mécanisme de déplacement (120).
